# EUROPEAN PATENT APPLICATION

(11) **EP 4 545 514 A1**
(43) Date of publication of application: **30.04.2025**
(21) Application number: 23831201.1
(22) Date of filing: 20.06.2023
(51) Int. Cl.: C07C 327/22, C07C 321/20, C07C 323/64, C07C 327/26, C08G 18/62, C08K 5/372, C08L 75/04, C08L 101/00, C09J 11/06, C09J 201/00

(54) **COMPOUND, ADDITIVE, PLASTICIZER, CURABLE COMPOSITION, ADHESIVE, CURED PRODUCT AND TACKIFIER**

(30) Priority: 27.06.2022 JP 2022102495; 29.11.2022 JP 2022190722
(71) Applicant: Mitsui Chemicals, Inc., Tokyo 104-0028 (JP)
(72) Inventor: TSUKADA, Yuichi, Sodegaura-shi, Chiba 299-0265 (JP); YONEDA, Shuhei, Sodegaura-shi, Chiba 299-0265 (JP)
(74) Representative: Hoffmann Eitle
(86) International application number: PCT/JP2023/022730
(87) International publication number: WO 2024/004755

(57) **Abstract**

A compound is represented by the general formula (1).
General formula (1);
Chem. 1

A-(SXR)ₙ (1)

(In the formula (1), the A represents an n-valent organic group including a sulfur atom. The n represents an integer of 3 or more. The S represents a sulfur atom. The X represents a single bond or a carbonyl group. The Xs may be the same or different from each other. The R represents an aliphatic hydrocarbon group, an aromatic hydrocarbon group, or an araliphatic hydrocarbon group. The Rs may be the same or different from each other.)

## Description

### TECHNICAL FIELD

The present invention relates to a compound, an additive, a plasticizer, a curable composition, an adhesive, a cured product, and a pressure-sensitive adhesive.

### BACKGROUND ART

Optical resin used as an adhesive and a pressure-sensitive adhesive in various optical fields has a relatively high refractive index.

Optical resin may be required to have flexibility depending on the use. In light thereof, it is considered to add a plasticizer to the optical resin. As the plasticizer, for example, dioctyl phthalate (DOP) has been known. Furthermore, as the plasticizer, for example, a dibenzoate of 2-mercaptoethylsulfide has been known (for example, see Patent Document 1 (Example 11)).

### Citation List

### Patent Document

Patent Document 1: Japanese Unexamined Patent Publication No. S61-019654

### SUMMARY OF THE INVENTION

### PROBLEM TO BE SOLVED BY THE INVENTION

When the above-described plasticizer is added to the optical resin, the crystallinity of the optical resin decreases while the flexibility thereof improves. However, when the crystallinity of the optical resin decreases, the refractive index of the optical resin decreases. Furthermore, for example, when the refractive index of the optical resin is not decreased by the plasticizer, the crystallinity of the optical resin does not sufficiently decrease. Thus, the flexibility may not sufficiently improve.

The present invention provides a compound, an additive, a plasticizer, a curable composition, an adhesive, a cured product, and a pressure-sensitive adhesive to produce a cured product having both a refractive index and flexibility.

### MEANS FOR SOLVING THE PROBLEM

The present invention [1] includes a compound represented by the following general (1). General Formula (1);
[Chem. 1]
Chem. 1

A-(SXR)n (1)

(In the formula (1), the A represents an n-valent organic group including a sulfur atom. The n represents an integer of 3 or more. The S represents a sulfur atom. The X represents a single bond or a carbonyl group. The Xs may be the same or different from each other. The R represents an aliphatic hydrocarbon group, an aromatic hydrocarbon group, or an araliphatic hydrocarbon group. The Rs may be the same or different from each other.)

The present invention [2] includes the compound described in the above-described [1], wherein the A represents an organic group including a sulfur atom and a main-group-element atom (other than a sulfur atom and a hydrogen atom), wherein in the A, a ratio of the number of the sulfur atoms is more than 20% with respect to a sum of the number of the sulfur atoms and the number of the main-group-element atoms (other than a sulfur atom and a hydrogen atom).

The present invention [3] includes the compound described in the above-described [1] or [2], being represented by the following general formula (2) or the following general formula (3). 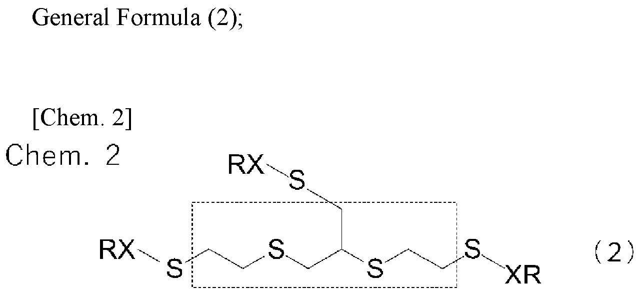 (In the formula (2), the S, X, and R represent the same meanings as the S, X, and R of the formula (1). In the formula (2), a portion surrounded by a dotted line represents an organic group A (n=3) of the formula (1).) 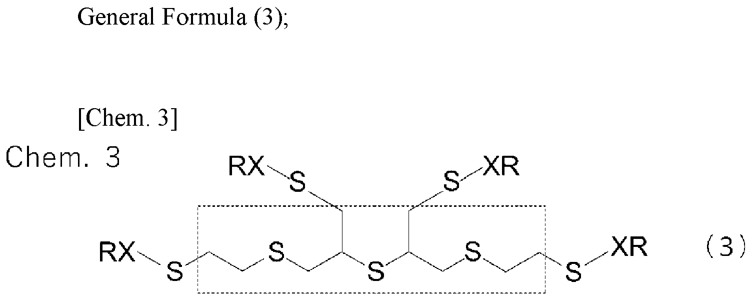 (In the formula (3), the S, X, and R represent the same meanings as the S, X, and R of the formula (1). In the formula (3), a portion surrounded by a dotted line represents an organic group A (n=4) of the formula (1).)

The present invention [4] includes the compound described in any one of the above-described [1] to [3], wherein the R represents a methyl group, a phenyl group, or a benzyl group.

The present invention [5] includes the compound described in any one of the above-described [1] to [3], wherein the X represents a carbonyl group, and the R represents an araliphatic hydrocarbon group.

The present invention [6] includes an additive including: the compound described in any one of the above-described [1] to [5].

The present invention [7] includes a plasticizer including: the compound described in any one of the above-described [1] to [5].

The present invention [8] includes a curable composition including the plasticizer described in the above-described [7] and a curable compound.

The present invention [9] includes the curable composition described in the above-described [8], further including: at least one additive selected from the group consisting of an ultraviolet absorber, a light-resistant stabilizer, and an antioxidant, wherein a ratio of the additive is 10×10⁻⁶ parts by mass or more and 10000×10⁻⁶ parts by mass or less with respect to 1 part by mass of a total amount of the curable composition.

The present invention [10] includes an adhesive including: the curable composition described in the above-described [8] or [9].

The present invention [11] includes a cured product including: the plasticizer described in the above-described [7] and a cured resin.

The present invention [12] includes the cured product described in the above-described [11], having a refractive index of 1.60 or more and a tensile storage elastic modulus of 100 MPa or less.

The present invention [13] includes the cured product described in the above-described [11] or [12], further including: at least one additive selected from the group consisting of an ultraviolet absorber, a light-resistant stabilizer, and an antioxidant, wherein a ratio of the additive is 10×10⁻⁶ parts by mass or more and 10000×10⁻⁶ parts by mass or less with respect to 1 part by mass of a total amount of the cured product.

The present invention [14] includes the cured product described in any one of the above-described [11] to [13], wherein the cured resin includes a reaction product containing a main agent containing an acrylic polyol and a curing agent containing a polyisocyanate.

The present invention [15] includes the cured product described in any one of the above-described [11] to [13], wherein the cured resin includes a cured acrylic resin.

The present invention [16] includes a pressure-sensitive adhesive including: the cured product described in any one of the above-described [11] to [15].

### EFFECTS OF THE INVENTION

According to the compound, additive, plasticizer, curable composition, and adhesive of the present invention, a cured product having both a refractive index and flexibility can be produced.

The cured product and pressure-sensitive adhesive of the present invention include the above-described compound, and thus have both a refractive index and flexibility.

### DESCRIPTION OF THE EMBODIMENTS

### 1. Compound

### (1) Structure of Compound

The compound of the present invention is represented by the following general formula (1). 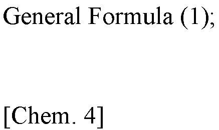 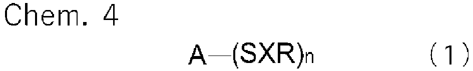 (In the formula (1), the A represents an n-valent organic group including a sulfur atom. The n represents an integer of 3 or more. The S represents a sulfur atom. The X represents a single bond or a carbonyl group. The Xs may be the same or different from each other. The R represents an aliphatic hydrocarbon group, an aromatic hydrocarbon group, or an araliphatic hydrocarbon group. The Rs may be the same or different from each other.)

### (2) A in Formula

In the above-described formula (1), the A represents an n-valent organic group including a sulfur atom. Preferably, the A represents an organic group including a sulfur atom and a main-group element atom (other than a sulfur atom and a hydrogen atom).

In the main-group-element atoms (other than a sulfur atom and a hydrogen atom), the main-group elements (other than sulfur and hydrogen) represent an element having an atomic number of 2 to 15, an element having an atomic number of 17 to 20, an element having an atomic number of 31 to 38, an element having an atomic number of 49 to 56, and an element having an atomic number of 81 to 88. As the main-group element (other than sulfur and hydrogen), preferably, an element having an atomic number of 2 to 15 is used, more preferably, an element having an atomic number of 6 to 9 is used, more specifically, carbon, nitrogen, oxygen, and fluorine are used. These can be used alone or in combination of two or more.

As the main-group elements (other than sulfur and hydrogen), even more preferably, carbon and oxygen are used, particularly preferably, carbon is used. In view of obtaining the refractive index and flexibility with good balance therebetween, more preferably, the A represents an organic group including a sulfur atom and a carbon atom, even more preferably, the A represents an organic group consisting of a sulfur atom and a carbon atom.

The n is a valence of the A. The n represents an integer of 3 or more. The n preferably represents an integer of 3 or more and 8 or less. The n more preferably represents an integer of 3 or more and 6 or less. The n even more preferably represents an integer of 3 or 4.

Examples of an n-valent A include a remaining group of an n-functional thiol. More specifically, examples of the A include a remaining group obtained by excluding the mercapto group from the trifunctional or more sulfur-containing polythiol (hereinafter, referred to as a trifunctional or more sulfur-containing polythiol remaining group).

The trifunctional or more sulfur-containing polythiol is an organic compound including 3 or more mercapto groups in one molecule and also including 1 or more (preferably, 2 or 3) sulfur atom(s) in addition to the mercapto groups in one molecule. Examples of the trifunctional or more sulfur-containing polythiol include a sulfur-containing trithiol, a sulfur-containing tetrathiol, a sulfur-containing pentathiol, a sulfur-containing hexathiol, and a sulfur-containing octathiol.

The sulfur-containing trithiol is a trifunctional thiol containing a sulfur atom in addition to the mercapto group. Examples of the sulfur-containing trithiol include 1,2,3-tris(mercaptomethylthio)propane, 1,2,3-tris(2-mercaptoethylthio)propane, 1,2,3-tris(3-mercaptopropylthio)propane, 4-mercaptomethyl-1,8-dimercapto-3,6-dithiaoctane (GST), 2,2-bis(mercaptomethylthio)ethanethiol, 3-mercaptomethylthio-1,7-dimercapto-2,6-dithiaheptane, 3-mercaptomethylthio-1,6-dimercapto-2,5-dithiahexane, 4,6-bis[4-(6-mercaptomethylthio)-1,3-dithianylthio]-6-[4-(6-mercaptomethylthio)-1,3-dithianylthio]-1,3-dithiane, tris(mercaptomethylthio)methane, tris(mercaptoethylthio)methane, 2,4,6-tris(mercaptomethylthio)-1,3,5-trithiacyclohexane, tris[(4-mercaptomethyl-2,5-dithiacyclohexyl-1-yl)methylthio]methane, 4-mercaptomethyl-2-(2,3-dimercaptopropylthio)-1,3-dithiacyclopentane, and 4-mercaptomethyl-2-(1,3-dimercapto-2-propylthio)-1,3-dithiacyclopentane.

The sulfur-containing tetrathiol is a tetrafunctional thiol containing a sulfur atom in addition to the mercapto group. Examples of the sulfur-containing tetrathiol include 5,7-dimercaptomethyl-1,11-dimercapto-3,6,9-trithiaundecane (FSH), 4,7-dimercaptomethyl-1,11-dimercapto-3,6,9-trithiaundecane, 4,8-dimercaptomethyl-1,11-dimercapto-3,6,9-trithiaundecane, tetrakis(mercaptomethylthiomethyl)methane, tetrakis(2-mercaptoethylthiomethyl)methane, tetrakis(3-mercaptopropylthiomethyl)methane, bis(2,3-dimercaptopropyl)sulfide, thiodipropionic acid bis(2,3-dimercaptopropylester), dithiodiglycolic acid bis(2,3-dimercaptopropylester), thiodipropionic acid bis(2,3-dimercaptopropylester), dithiodipropionic acid bis(2,3-dimercaptopropylester), 1,1,3,3-tetrakis(mercaptomethylthio)propane, 1,1,2,2-tetrakis(mercaptomethylthio)ethane, 1,1,5,5-tetrakis(mercaptomethylthio)-3-thiapentane, 1,1,6,6-tetrakis(mercaptomethylthio)-3,4-dithiahexane, 2,5-bis(4,4-bis(mercaptomethylthio)-2-thiabutyl)-1,4-dithiane, 2,2-bis(mercaptomethylthio)-1,3-propanedithiol, 3,6-bis(mercaptomethylthio)-1,9-dimercapto-2,5,8-trithianonan, 4-[3,5-bis(mercaptomethylthio)-7-mercapto-2,6-dithiaheptylthio]-6-mercaptomethylthio-1,3-dithiane, 1,1-bis[4-(6-mercaptomethylthio)-1,3-dithianylthio]-1,3-bis(mercaptomethylthio)propane, 3-[2-(1,3-dithietanyl)]methyl-7,9-bis(mercaptomethylthio)-1,11-dimercapto-2,4,6,10-tetrathiaundecane, 4-[3,4-bis(mercaptomethylthio)-6-mercapto-2,5-dithiahexylthio]-5-mercaptomethylthio-1,3-dithiolane, 2-[3,4-bis(mercaptomethylthio)-6-mercapto-2,5-dithiahexylthio]mercaptomethylthiomethyl-1,3-dithiethane, 4-{1-[2-(1,3-dithietanyl)]-3-mercapto-2-thiapropylthiol-5-[1,2-bis(mercaptomethylthio)-4-mercapto-3-thiabutylthio]-1,3-dithiolane, 1,1,5,5-tetrakis(mercaptomethylthio)-2,4-dithiapentane, and 1,1,3,3-tetrakis(mercaptomethylthio)-2-thiapropane.

The sulfur-containing pentathiol is a penta-functional thiol containing a sulfur atom in addition to the mercapto group. Examples of the sulfur-containing pentathiol include 1-[4-(6-mercaptomethylthio)-1,3-dithianylthio]-3-[2,2-bis(mercaptomethylthio)ethyl]-7,9-bis(mercaptomethylthio)-2,4,6,10-tetrathiaundecane and bis[4,4-bis(mercaptomethylthio)-1,3-dithiabutyl]-(mercaptomethylthio)methane.

The sulfur-containing hexathiol is a hexa-functional thiol containing a sulfur atom in addition to the mercapto group. Examples of the sulfur-containing hexathiol include 1,1,9,9-tetrakis(mercaptomethylthio)-5-(3,3-bis(mercaptomethylthio)-1-thiapropyl)3,7-dithianonane, tris(2,2-bis(mercaptomethylthio)ethyl)methane, tris(4,4-bis(mercaptomethylthio)-2-thiabutyl)methane, 3,5,9,11-tetrakis(mercaptomethylthio)-1,13-dimercapto-2,6,8,12-tetrathiatridecane, 3,4,8,9-tetrakis(mercaptomethylthio)-1,11-dimercapto-2,5,7,10-tetrathiaundecane, 4,6-bis[3,5-bis(mercaptomethylthio)-7-mercapto-2,6-dithiaheptylthio]-1,3-dithiane, 3-[2-(1,3-dithietanyl)]methyl-7,9,13,15-tetrakis(mercaptomethylthio)-1,17-dimercapto-2,4,6,10,12,16-hexathiaheptadecane, 4-[3,4,8,9-tetrakis(mercaptomethylthio)-11-mercapto-2,5,7,10-tetrathiaundecyl]-5-mercaptomethylthio-1,3-dithiolane, 4,5-bis[3,4-bis(mercaptomethylthio)-6-mercapto-2,5-dithiahexylthio]-1,3-dithiolane, 4-[3-bis(mercaptomethylthio)methyl-5,6-bis(mercaptomethylthio)-8-mercapto-2,4,7-trithiaoctyl]-5-mercaptomethylthio-1,3-dithiolane, 2-{bis[3,4-bis(mercaptomethylthio)-6-mercapto-2,5-dithiahexylthio]methyl}-1,3-dithiethane, 2-[3,4,8,9-tetrakis(mercaptomethylthio)-11-mercapto-2,5,7,10-tetrathiaundecylthio]mercaptomethylthiomethyl-1,3-dithiethane, 2-[3-bis(mercaptomethylthio)methyl-5,6-bis(mercaptomethylthio)-8-mercapto-2,4,7-trithiaoctyl]mercaptomethylthiomethyl-1,3-dithiethane, tris[4,4-bis(mercaptomethylthio)-1,3-dithiabutyl]methane, tris[2,2-bis(mercaptomethylthio)-2-thiapropyl]methane, tris[4,4-bis(mercaptomethylthio)-3-thiabutyl]methane, and 2,4,6-tris[3,3-bis(mercaptomethylthio)-2-thiapropyl]-1,3,5-trithiacyclohexane.

The sulfur-containing octathiol is an octa-functional thiol containing a sulfur atom in addition to the mercapto group. Examples of the sulfur-containing octathiol include tetrakis(4,4-bis(mercaptomethylthio)-2-thiabutyl)methane, 3,5,9,11,15,17-hexakis(mercaptomethylthio)-1,19-dimercapto-2,6,8,12,14,18-hexathianonadecane, 9-(2,2-bis(mercaptomethylthio)ethyl)-3,5,13,15-tetrakis(mercaptomethylthio)-1,17-dimercapto-2,6,8,10,12,16-hexathiaheptadecane, tetrakis(2,2-bis(mercaptomethylthio)ethyl)methane, 3,4,8,9,13,14-hexakis(mercaptomethylthio)-1,16-dimercapto-2,5,7,10,12,15-hexathiahexadecane, 8-[bis(mercaptomethylthio)methyl]-3,4,12,13-tetrakis(mercaptomethylthio)-1,15-dimercapto-2,5,7,9,11,14-hexathiapentadecane, and tetrakis[3,3-bis(mercaptomethylthio)-2-thiapropyl]methane.

As the trifunctional or more sulfur-containing polythiol remaining group, preferably, a trifunctional to hexafunctional sulfur-containing polythiol remaining group is used, more preferably, a trifunctional to tetrafunctional sulfur-containing polythiol remaining group is used, even more preferably, a trifunctional sulfur-containing polythiol remaining group is used.

In other words, in the general formula (1), as the A, preferably, a trifunctional to hexafunctional sulfur-containing polythiol remaining group is used, more preferably, a trifunctional to tetrafunctional sulfur-containing polythiol remaining group is used, even more preferably, a trifunctional sulfur-containing polythiol remaining group is used.

In other words, as the A, preferably, a trivalent to hexavalent organic group including 1 or more (preferably, 2 or 3) sulfur atom(s) is used. As the A, more preferably, a trivalent organic group including 1 or more (preferably, 2 or 3) sulfur atom(s) and a tetravalent organic group including a 1 or more (preferably, 2 or 3) sulfur atom(s) are used, even more preferably, a trivalent organic group including a sulfur atom is used.

In view of obtaining the refractive index and flexibility with good balance therebetween, preferably, the A contains sulfur atoms in a predetermined ratio or more. More specifically, in the A, the ratio of the number of sulfur atoms is, for example, more than 20%, preferably 21% or more, more preferably 22% or more with respect to the sum of the number of the sulfur atoms and the number of the main-group-element atoms (other than a sulfur atom and a hydrogen atom). Furthermore, in view of obtaining the refractive index and flexibility with good balance therebetween, the ratio of the number of the sulfur atoms is, for example, 80% or less, preferably 50% or less, more preferably 30% or less, even more preferably 25% or less with respect to the sum of the number of the sulfur atoms and the number of the main-group-element atoms (other than a sulfur atom and a hydrogen atom). The ratio of the number of the sulfur atoms with respect to the sum of the number of the sulfur atoms and the number of the main-group-element atoms (other than a sulfur atom and a hydrogen atom) is calculated by the following formula.

Ratio of Number of Sulfur Atoms (%) = Number of Sulfur Atoms/[Number of Sulfur Atoms + Number of Main-group-element Atoms (Other Than Sulfur Atom and Hydrogen Atom)]×100

As the trivalent organic group (organic group A (n=3)) including 1 or more (preferably 2 or 3, more preferably 2) sulfur atom(s), preferably, the above-described remaining group obtained by excluding the mercapto group from the sulfur-containing trithiol is used, more preferably, the remaining group (GST remaining group) obtained by excluding the mercapto group from 4-mercaptomethyl-1,8-dimercapto-3,6-dithiaoctane (GST) is used.

The remaining group (GST remaining group) obtained by excluding the mercapto group from 4-mercaptomethyl-1,8-dimercapto-3,6-dithiaoctane (GST) is, for example, represented by the following general formula (4). 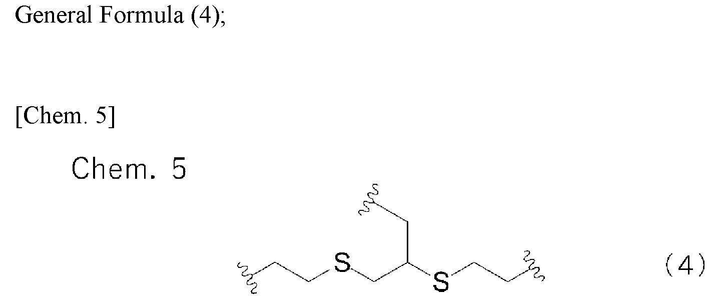 (In the formula (4), the wavy lines represent the position of the bond to the SXR group of the formula (1).)

When the A of the above-described formula (1) is the GST remaining group, a cured product having both a particularly excellent refractive index and particularly excellent flexibility is produced.

The GST remaining group is an organic group consisting of 2 sulfur atoms and 7 carbon atoms. In the GST remaining group, the ratio of the number of the sulfur atoms with respect to the sum of the number of the sulfur atoms and the number of the main-group-element atoms (other than a sulfur atom and a hydrogen atom) is approximately 22% (2/[2+7]×100).

As the tetravalent organic group (organic group A (n=4)) including 1 or more (preferably 2 or 3, more preferably 3) sulfur atom(s), preferably, the above-described remaining group obtained by excluding the mercapto group from the sulfur-containing tetrathiol is used, more preferably, the remaining group (FSH remaining group) obtained by excluding the mercapto group from 5,7-dimercaptomethyl-1,11-dimercapto-3,6,9-trithiaundecane (FSH) is used.

The remaining group (FSH remaining group) obtained by excluding the mercapto group from 5,7-dimercaptomethyl-1,11-dimercapto-3,6,9-trithiaundecane (FSH) is represented by, for example, the following general formula (5). 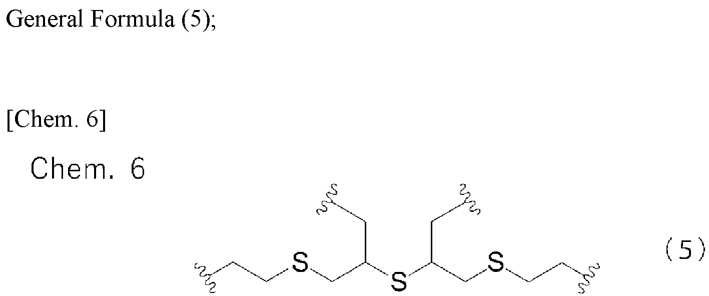 (In the formula (5), the wavy lines represent the position of the bond to the SXR group of the formula (1).)

When the A of the above-described formula (1) is the FSH remaining group, a cured product having both a particularly excellent refractive index and particularly excellent flexibility is produced.

The FSH remaining group is an organic group consisting of 3 sulfur atoms and 10 carbon atoms. In the FSH remaining group, the ratio of the number of sulfur atoms with respect to the sum of the number of the sulfur atoms and the number of the main-group-element atoms (other than a sulfur atom and a hydrogen atom) is approximately 23% (3/[3+10]×100).

In view of the refractive index and flexibility, as the A of the above-described formula (1), preferably, a GST remaining group and an FSH remaining group are used, more preferably, a GST remaining group is used.

### (3) S in Formula

In the above-described formula (1), the S represents a sulfur atom.

### (4) X in Formula

In the above-described formula (1), the X represents a single bond or a carbonyl group.

When the X represents a single bond, the S and R are directly bonded in the above-described formula (1). In other words, when the X represents a single bond, the SXR group of the above-described formula (1) represents an SR group.

When the X represents a carbonyl group, the S and R are indirectly bonded through a carbonyl group in the above-described formula (1). In other words, when the X represents a carbonyl group, the SXR group of the above-described formula (1) represents an S(C=O)R group.

The compound of the above-described formula (1) includes 3 or more SXR groups depending on the value of the n. Each X included in each SXR group may be the same or different from each other. Preferably, in the above-described formula (1), each X included in each SXR group is the same.

### (5) R in Formula

**In** the above-described formula (1), the R represents an aliphatic hydrocarbon group, an aromatic hydrocarbon group, or an araliphatic hydrocarbon group.

Examples of the aliphatic hydrocarbon group include an aliphatic hydrocarbon group having 1 to 20 carbon atom(s). More specifically, examples of the aliphatic hydrocarbon group include a straight-chain aliphatic hydrocarbon group having 1 to 20 carbon atom(s) and a cyclic aliphatic hydrocarbon group having 3 to 20 carbon atoms.

Examples of the straight-chain aliphatic hydrocarbon group having 1 to 20 carbon atom(s) include a straight-chain saturated aliphatic hydrocarbon group having 1 to 20 carbon atom(s) and a straight-chain unsaturated aliphatic hydrocarbon group having 1 to 20 carbon atom(s). Examples of the straight-chain saturated aliphatic hydrocarbon group having 1 to 20 carbon atom(s) include a methyl group, an ethyl group, an n-propyl group, an isopropyl group, an n-butyl group, a sec-butyl group, a tert-butyl group, a 2-butyl group, a 1-pentyl group, a 2-pentyl group, a 3-pentyl group, a 2-methyl-1-butyl group, an isopentyl group, a tert-pentyl group, a 3-methyl-2-butyl group, a neopentyl group, an n-hexyl group, a 4-methyl-2-pentyl group, a 1-heptyl group, a 3-heptyl group, a 1-octyl group, a 2-octyl group, a 2-ethyl-1-hexyl group, a 1,1-dimethyl-3,3-dimethylbutyl group, a 1-nonyl group, a 1-decyl group, an undecyl group, a dodecyl group, a tridecyl group, a tetradecyl group, a pentadecyl group, a hexadecyl group, a heptadecyl group, an octadecyl group, a nonadecyl group, and an eicosyl group. Examples of the straight-chain unsaturated aliphatic hydrocarbon group having 1 to 20 carbon atom(s) include a vinyl group and a 2-propenyl group. These can be used alone or in combination of two or more.

Examples of the cyclic aliphatic hydrocarbon group having 3 to 20 carbon atoms include a cyclic saturated aliphatic hydrocarbon group having 3 to 20 carbon atoms and a cyclic unsaturated aliphatic hydrocarbon group having 3 to 20 carbon atoms. Examples of the cyclic saturated aliphatic hydrocarbon group having 3 to 20 carbon atoms include a cyclopropyl group, a cyclobutyl group, a cyclopentyl group, a cyclohexyl group, a cycloheptyl group, a cyclooctyl group, a cyclononyl group, and a cyclodecyl group. Examples of the cyclic unsaturated aliphatic hydrocarbon group having 3 to 20 carbon atoms include a cyclopentenyl group and a cyclohexenyl group. These can be used alone or in combination of two or more.

Examples of the aromatic hydrocarbon group include an aromatic hydrocarbon group having 6 to 20 carbon atoms. Examples of the aromatic hydrocarbon group having 6 to 20 carbon atoms include a phenyl group, a 2-tolyl group, a 3-tolyl group, a 4-tolyl group, a 2,3-xylyl group, a 2,4-xylyl group, a 2,5-xylyl group, a 2,6-xylyl group, a 3,4-xylyl group, a 3,5-xylyl group, a 2,3,4-trimethyl phenyl group, a 3,4,5-trimethyl phenyl group, a 2,4,6-trimethyl phenyl group, a 2,3,4,5-tetramethyl phenyl group, a 2,3,4,6-tetramethyl phenyl group, a 2-ethyl phenyl group, a 3-ethyl phenyl group, a 4-ethyl phenyl group, a 1-naphthyl group, and a 2-naphthyl group. These can be used alone or in combination of two or more.

Examples of the araliphatic hydrocarbon group include an araliphatic hydrocarbon group having 7 to 20 carbon atoms. Examples of the araliphatic hydrocarbon group having 7 to 20 carbon atoms include a benzyl group, a 1-phenyl ethyl group, a 2-phenyl ethyl group, a 1-phenylpropyl, a 2-phenylpropyl, a 3-phenylpropyl, an o-methylbenzyl, a m-methylbenzyl, a p-methylbenzyl, an o-ethylbenzyl, a m-ethylbenzyl, a p-ethylbenzyl, an o-isopropylbenzyl, an m-isopropylbenzyl, a p-isopropylbenzyl, a 2,3,4-trimethylbenzyl, a 3,4,5-trimethylbenzyl, and a 2,4,6-trimethylbenzyl. These can be used alone or in combination of two or more.

The aliphatic hydrocarbon group, the aromatic hydrocarbon group, and the araliphatic hydrocarbon group can include a substituent. Examples of the substituent include a halogeno group, a cyano group, an amino group, a carboxy group, a sulfonyl group, and an alkoxy group. These can be used alone or in combination of two or more. The number of the substituents is appropriately set depending on the purpose and use. The position of the substitution is appropriately set depending on the purpose and use.

The compound of the above-described formula (1) includes 3 or more SXR groups depending on the value of the n. The R included in Each SXR group may be the same or different from each other. Preferably, one type selected from the group consisting of an aliphatic hydrocarbon group, an aromatic hydrocarbon group, and an araliphatic hydrocarbon group is used alone. In other words, preferably, the R included in each SXR group is the same in the above-described formula (1).

When the R represents an aliphatic hydrocarbon group, preferably, a straight-chain aliphatic hydrocarbon group having 1 to 10 carbon atom(s) is used, more preferably, a straight-chain aliphatic hydrocarbon group having 1 to 4 carbon atom(s) is used, even more preferably, a straight-chain aliphatic hydrocarbon group having 1 to 2 carbon atom(s) is used, particularly preferably, a methyl group is used.

When the R represents an aromatic hydrocarbon group, preferably, an aromatic hydrocarbon group having 6 to 10 carbon atoms is used, more preferably, an aromatic hydrocarbon group having 6 to 8 carbon atoms is used, even more preferably, a phenyl group is used.

When the R represents an araliphatic hydrocarbon group, preferably, an araliphatic hydrocarbon group having 7 to 15 carbon atoms is used, more preferably, an araliphatic hydrocarbon group having 7 to 10 carbon atoms is used, even more preferably, a benzyl group and a 2-phenyl ethyl group are used.

In view of obtaining a cured product having the refractive index and flexibility with good balance, the R preferably represents a methyl group, a phenyl group, or a benzyl group in the above-described formula (1). In other words, when the R is a methyl group, a phenyl group, or a benzyl group, a cured product having a particularly excellent refractive index and a particularly excellent flexibility is obtained.

In view of the refractive index, even more preferably, the R represents a phenyl group or a benzyl group. Furthermore, in view of the flexibility, even more preferably, the R represents a methyl group.

Furthermore, in view of obtaining excellent weather resistance in addition to the excellent refractive index and the excellent flexibility, in the above-described formula (1), preferably, the X represents a carbonyl group and also the R represents an araliphatic hydrocarbon group and/or an aliphatic hydrocarbon group, more preferably, the X represents a carbonyl group and also the R represents an araliphatic hydrocarbon group. In other words, when the X is a carbonyl group and also the R is an araliphatic hydrocarbon group, a cured product having all of a particularly excellent refractive index, particularly excellent flexibility, and particularly excellent weather resistance is be obtained.

In view of the refractive index, even more preferably, the X represents a carbonyl group and also the R represents a benzyl group. Furthermore, in view of the flexibility and weather resistance, even more preferably, the X represents a carbonyl group and also the R represents a 2-phenyl ethyl group and/or a methyl group, particularly preferably, the X represents a carbonyl group and also the R represents a 2-phenyl ethyl group.

### (6) Specific Example

As the compound represented by the above-described formula (1), preferably, a compound including a GST remaining group represented by the A in the formula (1) is used. The compound including a GST remaining group represented by the A in the formula (1) is represented by, for example, the following general formula (2). 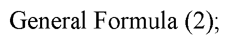 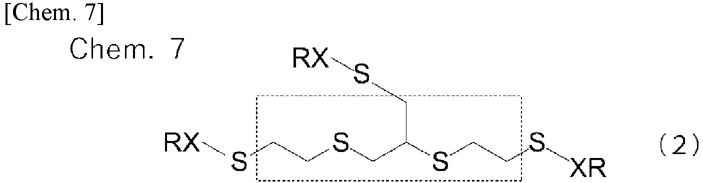 (In the formula (2), the S, X and R have the same meanings as the S, X and R of the formula (1). In the formula (2), the portion surrounded by the dotted line represents an organic group A (n=3) of the formula (1).)

According to the compound represented by the above-described formula (2), a cured product having both a particularly excellent refractive index and particularly excellent flexibility is obtained.

More specifically, examples of the compound represented by the above-described formula (2) include 4-benzylthiomethyl-1,8-bis benzylthio-3,6-dithiaoctane (Bn-GST). In the Bn-GST, the X of the formula (2) represents a single bond and the R represents a benzyl group.

Furthermore, more specifically, examples of the compound represented by the above-described formula (2) also include 4-benzoylthiomethyl-1,8-bis benzoylthio-3,6-dithiaoctane (Bz-GST). In the Bz-GST, the X of the formula (2) represents a carbonyl group and the R represents a phenyl group.

Furthermore, more specifically, examples of the compound represented by the above-described formula (2) also include 4- acetylthiomethyl-1,8-bis acetylthio-3,6-dithiaoctane (Ac-GST). In the Ac-GST, the X of the formula (2) represents a carbonyl group and the R represents a methyl group.

Furthermore, more specifically, examples of the compound represented by the above-described formula (2) include 4-phenyl acetylthiomethyl-1,8-bisphenyl acetylthio-3,6-dithiaoctane (PA-GST). In the PA-GST, the X of the formula (2) represents a carbonyl group and the R represents a benzyl group.

Furthermore, more specifically, examples of the compound represented by the above-described formula (2) also include 4-(3-phenyl propionyl) thiomethyl-1,8-bis(3-phenyl propionyl)thio-3,6-dithiaoctane (PP-GST). In the PP-GST, the X of the formula (2) represents a carbonyl group and the R represents a 2-phenyl ethyl group.

Furthermore, as the compound represented by the above-described formula (1), preferably, a compound including an FSH remaining group represented by the A in the formula (1) is used. The compound including an FSH remaining group represented by the A in the formula (1) is represented, for example, by the following general formula (3).

### General Formula (3);

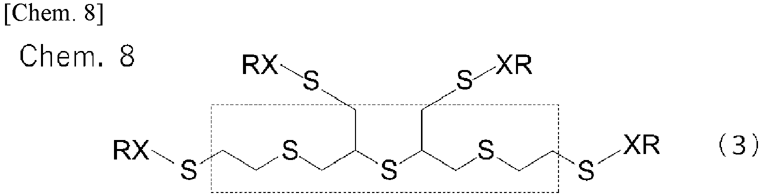

(In the formula (3), the S, X and R have the same meanings of the S, X and R of the formula (1). In the formula (3), the portion surrounded by the dotted line represents an organic group A(n=4) of the formula (1).)

According to the compound represented by the above-described formula (3), a cured product having both a particularly excellent refractive index and particularly excellent flexibility is obtained.

More specifically, examples of the compound represented by the above-described formula (3) include 5,7-bis(benzoylmercaptomethyl)-1,11-bis(benzoylmercapto)-3,6,9-trithiaundecane (Bz-FSH). In the Bz-FSH, the X of the formula (3) represents a single bond and the R represents a benzyl group.

### 2. Method of Producing Compound

### (1) Material of Compound

The above-described compound is produced, for example, by the reaction between the above-described trifunctional or more sulfur-containing polythiol and a modifier.

The modifier is a compound that modifies the mercapto group of the above-described trifunctional or more sulfur-containing polythiol to the SXR group in the above-described formula (1).

Examples of the modifier include a compound represented by the following general formula (6).
General Formula (6);
[Chem. 9] Chem. 9

Y-XR (6)

(In the formula (6), the X and R have the same meanings as the X and R of the formula (1). The Y represents a halogen or a hydroxyl group.)

In the above-described formula (6), the X and R represent the same meanings as the X and R of the formula (1). The Y represents a halogen or a hydroxyl group. Examples of the halogen include fluorine, chlorine, bromine, and iodine. As the halogen, preferably, chlorine and bromine are used.

When the Y is a halogen and the X is a single bond in the above-described formula (6), examples of the modifier include halogenated hydrocarbon, halogenated alkyl, halogenated aryl, and halogenated aralkyl. Examples of the halogenated alkyl include methyl fluoride, methyl chloride, methyl bromide, methyl iodide, ethyl fluoride, ethyl chloride, ethyl bromide, and ethyl iodide. Examples of the halogenated aryl include phenyl fluoride, phenyl chloride, phenyl bromide, and phenyl iodide. Examples of the halogenated aralkyl include benzyl fluoride, benzyl chloride, benzyl bromide (benzyl bromide), and benzyl iodide. These can be used alone or in combination of two or more. As the above-described modifier, preferably, halogenated aralkyl is used, more preferably, benzyl bromide (benzyl bromide) is used.

When the Y is a halogen and X is a carbonyl group in the above-described formula (6), examples of the modifier include halogenated acyl. Examples of halogenated acyl include acetyl fluoride, acetyl chloride, acetyl bromide, acetyl iodide, benzoyl fluoride, benzoyl chloride, benzoyl bromide, and benzoyl iodide. These can be used alone or in combination of two or more. As the above-described modifier, preferably, benzoyl chloride is used.

When the Y is a hydroxyl group and the X is a carbonyl group in the above-described formula (6), examples of the modifier include carboxylic acid. Examples of the carboxylic acid include monocarboxylic acid and an anhydride thereof. Examples of the monocarboxylic acid include an aliphatic monocarboxylic acid, an aromatic monocarboxylic acid, and an araliphatic monocarboxylic acid. Examples of an aliphatic monocarboxylic acid include acetic acid, propionic acid, butyric acid, caproic acid, octylic acid, lauric acid, myristic acid, palmitic acid, stearic acid, 2-ethyl hexanoic acid, cyclohexane carboxylic acid, and cyclopentane carboxylic acid. Examples of the aromatic monocarboxylic acid include benzoic acid and toluic acid. Examples of the araliphatic monocarboxylic acid include diphenyl acetic acid. These can be used alone or in combination of two or more. As the above-described modifier, preferably, an aliphatic monocarboxylic acid and an anhydride thereof are used, more preferably, acetic acid and an anhydride thereof are used, even more preferably, acetic anhydride is used.

### (2) Mixing Ratio and Conditions for Reaction

The trifunctional or more sulfur-containing polythiol and the modifier are mixed together in an appropriate ratio and are reacted under appropriate conditions. The mixing ratio of the trifunctional or more sulfur-containing polythiol and the modifier is selected according to the type of the trifunctional or more sulfur-containing polythiol and the type of the modifier. Examples of the reaction between the trifunctional or more sulfur-containing polythiol and the modifier include a nucleophilic substitution reaction, a nucleophilic acylation reaction, a cross-coupling reaction, and a dehydration-condensation reaction.

For example, when the modifier includes halogenated hydrocarbon, the trifunctional or more sulfur-containing polythiol and the modifier are subjected to a nucleophilic substitution reaction in the presence of a known basic compound to produce a compound of the above-described formula (1). More specifically, the basic compound desorbs the proton of the trifunctional or more sulfur-containing polythiol to produce a nucleophilic agent. Then, the nucleophilic agent derived from the trifunctional or more sulfur-containing polythiol and halogenated hydrocarbon are subjected to a nucleophilic substitution reaction. Examples of the basic compound include a metal alcoholate and an amine compound. In such a reaction, the mixing ratio of the trifunctional or more sulfur-containing polythiol to the modifier is adjusted based on the equivalent ratio of the halogen atoms in the modifier (halogenated hydrocarbon) with respect to the mercaptos group in the sulfur-containing polythiol. The equivalent ratio (halogen atoms/mercapto groups) of the halogen atom in the modifier (halogenated hydrocarbon) is, for example, 0.9 or more, preferably 1.0 or more, more preferably 1.01 or more with respect to the mercapto groups in the sulfur-containing polythiol. Furthermore, the equivalent ratio (halogen atoms/mercapto groups) of the halogen atoms in the modifier (halogenated hydrocarbon) is, for example, 5.0 or less, preferably 3.0 or less, more preferably 1.5 or less with respect to the mercapto groups in the sulfur-containing polythiol.

When the modifier includes halogenated hydrocarbon, the reaction conditions for the nucleophilic substitution reaction are appropriately selected depending on the type of the trifunctional or more sulfur-containing polythiol and the type of the modifier. For example, the reaction temperature is, for example, -20°C or more, preferably -10°C or more. Furthermore, the reaction temperature is, for example, 50°C or less, preferably 30°C or less. Furthermore, the reaction time is, for example, 3 hours or more, preferably 6 hours or more. Furthermore, the reaction time is, for example, 48 hours or less, preferably 24 hours or less. Furthermore, the trifunctional or more sulfur-containing polythiol and the modifier may be reacted under no solvent or under a known solvent. The type of the solvent and the adding amount thereof are appropriately set. Furthermore, the trifunctional or more sulfur-containing polythiol and the modifier may be reacted under no catalyst or under a known catalyst. The type of the catalyst and the adding amount thereof are appropriately set.

Furthermore, for example, when the modifier includes halogenated acyl, the trifunctional or more sulfur-containing polythiol and the modifier are subjected to a nucleophilic acylation reaction in the presence of the above-described basic compound to produce a compound of the above-described formula (1). More specifically, the basic compound desorbs the proton of the trifunctional or more sulfur-containing polythiol to produce a nucleophilic agent. Then, the nucleophilic agent derived from the trifunctional or more sulfur-containing polythiol and the halogenated acyl are subjected to a nucleophilic acylation reaction. In such a reaction, the mixing ratio of the trifunctional or more sulfur-containing polythiol to the modifier is adjusted based on the equivalent ratio of the halogen atoms in the modifier (halogenated acyl) with respect to the mercapto groups in the sulfur-containing polythiol. The equivalent ratio (halogen atoms/mercapto groups) of the halogen atoms in the modifier (halogenated acyl) is, for example, 0.9 or more, preferably 1.0 or more, more preferably 1.05 or more with respect to the mercapto groups in the sulfur-containing polythiol. Furthermore, the equivalent ratio (halogen atoms/mercapto groups) of the halogen atom in the modifier (halogenated acyl) is, for example, 5.0 or less, preferably 3.0 or less, more preferably 1.5 or less with respect to the mercapto groups in the sulfur-containing polythiol.

When the modifier includes halogenated acyl, the reaction conditions for the nucleophilic acylation reaction are appropriately selected depending on the type of the trifunctional or more sulfur-containing polythiol and the type of the modifier. For example, the reaction temperature is, for example, -20°C or more, preferably -10°C or more. Furthermore, the reaction temperature is, for example, 50°C or less, preferably 30°C or less. Furthermore, the reaction time is, for example, 3 hours or more, preferably 6 hours or more. Furthermore, the reaction time is, for example, 48 hours or less, preferably 24 hours or less. Furthermore, the trifunctional or more sulfur-containing polythiol and the modifier may be reacted under no solvent or under a known solvent. The type of the solvent and the adding amount thereof are appropriately set. Furthermore, the trifunctional or more sulfur-containing polythiol and the modifier may be reacted under no catalyst or under a known catalyst. The type of the catalyst and the adding amount thereof are appropriately set.

Furthermore, for example, when the modifier includes carboxylic acid, the trifunctional or more sulfur-containing polythiol and the modifier (carboxylic acid) are subjected to a dehydration-condensation reaction in the presence of the above-described basic compound to produce a compound of the above-described formula (1). In such a reaction, the mixing ratio of the trifunctional or more sulfur-containing polythiol to the modifier is adjusted based on the equivalent ratio of the carboxy groups in the modifier (carboxylic acid) with respect to the mercapto groups in the sulfur-containing polythiol. The equivalent ratio (carboxy groups/mercapto groups) of the carboxy group in the modifier (carboxylic acid) is, for example, 0.9 or more, preferably 1.0 or more, more preferably 1.05 or more with respect to the mercapto groups in the sulfur-containing polythiol. Furthermore, the equivalent ratio (carboxy groups/mercapto groups) of the carboxy groups in the modifier (carboxylic acid) is, for example, 5.0 or less, preferably 3.0 or less, more preferably 2.5 or less with respect to the mercapto groups in the sulfur-containing polythiol.

When the modifier includes carboxylic acid, the reaction conditions for the dehydration-condensation reaction are appropriately selected depending on the type of the trifunctional or more sulfur-containing polythiol and the type of the modifier. For example, the reaction temperature is, for example, -20°C or more, preferably -10°C or more. Furthermore, the reaction temperature is, for example, 50°C or less, preferably 30°C or less. Furthermore, the reaction time is, for example, 3 hours or more, preferably 6 hours or more. Furthermore, the reaction time is, for example, 48 hours or less, preferably 24 hours or less. Furthermore, the trifunctional or more sulfur-containing polythiol and the modifier may be reacted under no solvent or under a known solvent. The type of the solvent and the adding amount thereof are appropriately set. Furthermore, the trifunctional or more sulfur-containing polythiol and the modifier may be reacted under no catalyst or under a known catalyst. The type of the catalyst and the adding amount thereof are appropriately set.

Then, after the completion of the above-described reaction, as necessary, a known neutralizer is added to the reaction mixture to adjust the pH of the reaction mixture. Furthermore, as necessary, the reaction product is concentrated or isolated in a known method. Furthermore, as necessary, the reaction product is purified in a known method. In this manner, a compound represented by the above-described formula (1) is produced as a reaction product.

Furthermore, the modifier and the reaction method are not limited to the above. Examples of the reaction method include an ene-thiol reaction. Furthermore, examples of the modifier include a compound that can be subjected together with the above-described trifunctional or more sulfur-containing polythiol to an ene-thiol reaction. Examples of such a compound include a vinyl compound. Examples of the vinyl compound include styrene, methyl styrene, and butyl styrene. These can be used alone or in combination of two or more.

When a vinyl compound is used as the modifier, the trifunctional or more sulfur-containing polythiol and the modifier (vinyl compound) are subjected to an ene-thiol reaction in the presence of a known radical initiator to produce a compound of the above-described formula (1) (the X represents a single bond, and the R represents a hydrocarbon group derived from the vinyl compound).

In the ene-thiol reaction, the radical polymerization initiator pulls the hydrogen atom out of the mercapto group of the trifunctional or more sulfur-containing polythiol to produce a thiyl radical. The thiyl radical is radically added to the vinyl group of the vinyl compound to produce a carbo radical. The carbo radical pulls the hydrogen atom out of another mercapto group. In this manner, the alkylation of the mercapto group is completed. Furthermore, together with this, the radical chain reaction continues. As a result, a compound of the above-described formula (1) is obtained.

The radical polymerization initiator is not especially limited, and examples thereof include a known radical polymerization initiator. More specifically, examples of the radical polymerization initiator include a photo-radical polymerization initiator and a thermal radical polymerization initiator. Examples of the photo-radical polymerization initiator include 1-hydroxy cyclohexane-1-yl phenyl ketone. Examples of the thermal radical polymerization initiator include an organic peroxide and an azo compound. Examples of the organic peroxide include a benzoyl peroxide. Examples of the azo compound include azo bis isobutyronitrile. These can be used alone or in combination of two or more. The mixing ratio of the radical polymerization initiator is appropriately set depending on the type of the radical polymerization initiator.

In the ene-thiol reaction, the mixing ratio of the trifunctional or more sulfur-containing polythiol to the modifier is adjusted based on the equivalent ratio of the vinyl groups in the modifier (vinyl compound) with respect to the mercapto groups in the sulfur-containing polythiol. The equivalent ratio (vinyl groups/mercapto groups) of the vinyl groups in the modifier (vinyl compound) is, for example, 0.9 or more, preferably 1.0 or more, more preferably 1.01 or more with respect to the mercapto groups in the sulfur-containing polythiol. Furthermore, the equivalent ratio (vinyl groups/mercapto groups) of the vinyl groups in the modifier (vinyl compound) is, for example, 5.0 or less, preferably 3.0 or less, more preferably 1.5 or less with respect to the mercaptos group in the sulfur-containing polythiol.

The reaction conditions for the ene-thiol reaction are appropriately selected depending on the type of the trifunctional or more sulfur-containing polythiol and the type of the modifier. For example, the reaction temperature is, for example, -20°C or more, preferably -10°C or more. Furthermore, the reaction temperature is, for example, 150°C or less, preferably 100°C or less. Furthermore, the reaction time is, for example, 3 hours or more, preferably 6 hours or more. Furthermore, the reaction time is, for example, 48 hours or less, preferably 24 hours or less. Furthermore, the trifunctional or more sulfur-containing polythiol and the modifier may be reacted under no solvent or under a known solvent. The type of the solvent and the adding amount thereof are appropriately set. Furthermore, the trifunctional or more sulfur-containing polythiol and the modifier may be reacted under no catalyst or under a known catalyst. The type of the catalyst and the adding amount thereof are appropriately set.

Then, after the completion of the above-described reaction, as necessary, the reaction product is concentrated or isolated in a known method. Furthermore, as necessary, the reaction product is purified in a known method. In this manner, a compound represented by the above-described formula (1) is produced as a reaction product.

The above-described compound is a novel compound and has a specific structure represented by the above-described formula (1). Examples of the use of such a compound include an additive. In other words, the additive preferably contains the above-described compound. More specifically, examples of the additive include a plasticizer and a refractive index adjuster. Preferably, a plasticizer is used. That is to say, the plasticizer preferably contains the above-described compound. In other words, preferably, the use of the above-described compound is a plasticizer. According to the above-described compound, a cured product (described below) having both a refractive index and flexibility can be produced.

### 3. Curable Composition

### (1) Main Component

The curable composition contains a plasticizer and a curable compound as a main component. The main component is a component contained in a content ratio of a predetermined value or more with respect to the whole. The content ratio of the main component with respect to the whole is, for example, 90% by mass or more. **In** other words, the total amount of the solid content of the plasticizer and the curable compound is 90% by mass or more with respect to the total amount of the solid content of the curable composition.

The curable compound is an uncured compound, and is a compound that is cured by a known method to produce a cured resin (described below). The plasticizer is an additive that improves the flexibility of the cured resin (described below). The plasticizer and the curable compound are detailed below.

### (2) Plasticizer

The plasticizer contains a compound represented by the above-described formula (1). The plasticizer preferably consists of a compound represented by the above-described formula (1).

When the plasticizer contains a compound represented by the above-described formula (1), the plasticizer can improve the flexibility of the cured product (described below), and suppress the decrease in refractive index of the cured product (described below) or improve the refractive index.

### (3) Curable Compound

The curable compound is not especially limited, and examples thereof include a resin material capable of producing a cured resin (described below).

Examples of the cured resin, as described below, include a cured polyurethane resin, a cured polyolefin resin, a cured polyamine resin, a cured amide resin, a cured urea resin, a cured phenol resin, a cured epoxy resin, a cured acrylic resin, a cured melamine resin, and a cured alkyd resin. These can be used alone or in combination of two or more.

As the cured resin (described below), preferably, a cured polyurethane resin and a cured acrylic resin are used. Then, the curable compound is selected depending on the type of the cured resin (described below).

For example, when a cured polyurethane resin is used as the cured resin, for example, a polyurethane resin material is used as the curable compound. Furthermore, when a cured acrylic resin is used as the cured resin, for example, an acrylic resin material is used as the curable compound.

The polyurethane resin material and the acrylic resin material are detailed below.

### (3-1) Polyurethane Resin Material

The polyurethane resin material is an uncured (unreacted) resin composition for producing a cured polyurethane resin (described below). The polyurethane resin material includes, for example, a polyisocyanate and a polyol.

### (3-2) Polyisocyanate

The polyisocyanate has a plurality of isocyanate groups in one molecule. Examples of the polyisocyanate include a polyisocyanate monomer and a polyisocyanate derivative.

Examples of the polyisocyanate monomer include an aliphatic polyisocyanate, an aromatic polyisocyanate, and an araliphatic polyisocyanate.

Examples of the aliphatic polyisocyanate include ethylene diisocyanate, trimethylene diisocyanate, 1,4-tetramethylene diisocyanate, 1,5-pentamethylene diisocyanate, 1,6-hexamethylene diisocyanate, trimethylhexamethylene diisocyanate, and 2,6-diisocyanatemethyl caproate. These can be used alone or in combination of two or more.

Furthermore, examples of the aliphatic polyisocyanate monomer include an alicyclic polyisocyanate monomer. Examples of the alicyclic polyisocyanate monomer include 1,3-cyclopentanediisocyanate, 1,3-cyclopentene diisocyanate, 1,4-cyclohexane diisocyanate, 1,3-cyclohexane diisocyanate, 3-isocyanatomethyl-3,5,5-trimethyl cyclohexyl isocyanate, methylene bis(cyclohexyl isocyanate), methyl-2,4-cyclohexane diisocyanate, methyl-2,6-cyclohexane diisocyanate, norbornane diisocyanate, and bis(isocyanatomethyl) cyclohexane. These can be used alone or in combination of two or more.

Examples of the aromatic polyisocyanate include tolylene diisocyanate, phenylene diisocyanate, 4,4'-diphenyl diisocyanate, 1,5-naphthalene diisocyanate, diphenylmethane diisocyanate, 4,4'-toluidine diisocyanate, and 4,4'-diphenylether diisocyanate. These can be used alone or in combination of two or more.

Examples of the araliphatic polyisocyanate include xylylene diisocyanate, tetramethylxylylene diisocyanate, and ω,ω'-diisocyanate-1,4-diethylbenzene. These can be used alone or in combination of two or more.

The polyisocyanate derivative is derived from the above-described polyisocyanate monomer. Examples of the polyisocyanate monomer include an isocyanurate modified product, an iminooxadiazinedione modified product, a triol adduct, an allophanate modified product, a biuret modified product, a urea modified product, an oxadiazine trione modified product, a carbodiimide modified product, and a uretdione modified product, and a uretonimine modified product. These can be used alone or in combination of two or more. As the polyisocyanate derivative, preferably, an isocyanurate modified product is used.

These polyisocyanates can be used alone or in combination of two or more. The polyisocyanate preferably contains a polyisocyanate derivative, more preferably consists of a polyisocyanate derivative.

As the polyisocyanate derivative, in view of improving the refractive index and transparency, preferably, a polyisocyanate derivative (araliphatic polyisocyanate derivative) derived from the araliphatic polyisocyanate is used.

As the araliphatic polyisocyanate derivative, more preferably, a derivative of xylylene diisocyanate is used, even more preferably, a triol adduct of xylylene diisocyanate is used.

The average number of the isocyanate groups of the polyisocyanate is, for example, 2 or more, preferably 2.5 or more. Furthermore, the average number of the isocyanate groups of the polyisocyanate is, for example, 4 or less, preferably 3.5 or less.

The isocyanate group content (NCO%) of the polyisocyanate is, for example, 5% by mass or more, preferably 7% by mass or more. Furthermore, the isocyanate group content (NCO%) of the polyisocyanate is, for example, 30% by mass or less, preferably 25% by mass or less.

### (3-3) Polyol

The polyol includes, for example, a macropolyol. The macropolyol has 2 or more hydroxyl groups in one molecule. The macropolyol is an organic compound having a relatively high molecular weight. The macropolyol has a number average molecular weight of, for example, 400 or more and, for example, 20000 or less. The number average molecular weight can be calculated from the hydroxyl group equivalent and the average number of the hydroxyl groups in a known method. Furthermore, the number average molecular weight can be measured as a molecular weight in terms of polystyrene by gel permeation chromatograph (hereinafter, the same applies).

Examples of the macropolyol include a polyether polyol, a polyester polyol, a polycarbonate polyol, a polyurethane polyol, an epoxy polyol, a vegetable oil polyol, a polyolefin polyol, an acrylic polyol, and a vinyl monomer modified polyol. These macropolyols can be used alone or in combination of two or more.

The macropolyol has a number average molecular weight of, for example, more than 400, preferably 500 or more, more preferably 1000 or more. Furthermore, the macropolyol has a number average molecular weight of, for example, 20000 or less, preferably 15000 or less, more preferably 10000 or less, even more preferably 5000 or less.

The macropolyol has a hydroxyl group value of, for example, 5 mgKOH/g or more, preferably 10 mgKOH/g or more, more preferably 15 mgKOH/g or more, even more preferably 20 mgKOH/g or more. Furthermore, the macropolyol has a hydroxyl group value of, for example, 500 mgKOH/g or less, preferably 300 mgKOH/g or less, more preferably 200 mgKOH/g or less, even more preferably 100 mgKOH/g or less, particularly preferably 50 mgKOH/g or less. The hydroxyl group value is measured in conformity with the description in JIS K 1557-1 (2007) (hereinafter, the same applies).

As the macropolyol, preferably, an acrylic polyol is used.

Examples of the acrylic polyol include a copolymer of the acrylic material component. The acrylic material component contains, for example, a hydroxyl group-containing (meth)acrylate and a copolymerizable vinyl monomer.

The (meth)acrylate indicates acrylate and/or methacrylate. Furthermore, the copolymerizable vinyl monomer indicates a vinyl monomer copolymerizable with the hydroxyl group-containing (meth)acrylate.

The hydroxyl group-containing (meth)acrylate contains a hydroxyl group and an alkyl group. Examples of the alkyl group include an alkyl group having 1 to 4 carbon atom(s). Specifically, examples of the hydroxy alkyl (meth)acrylate include 2-hydroxy ethyl (meth)acrylate, hydroxy propyl (meth)acrylate, hydroxy butyl (meth)acrylate, and 2,2-dihydroxy methylbutyl (meth)acrylate. These can be used alone or in combination of two or more. As the hydroxyl group-containing (meth)acrylate, preferably, hydroxy alkyl (meth)acrylate is used, more preferably, 2-hydroxy ethyl (meth)acrylate is used, even more preferably, 2-hydroxy ethyl methacrylate is used.

Examples of the copolymerizable vinyl monomer include alkyl (meth)acrylate. The alkyl (meth)acrylate does not contain a hydroxyl group and contains an alkyl group. Examples of the alkyl group include an alkyl group having 1 to 4 carbon atom(s) and an alkyl group having 5 to 8 carbon atoms. Examples of the alkyl (meth)acrylate having an alkyl group having 1 to 4 carbon atom(s) include methyl (meth)acrylate, ethyl (meth)acrylate, propyl (meth)acrylate, isopropyl (meth)acrylate, butyl (meth)acrylate, isobutyl (meth)acrylate, s-butyl (meth)acrylate, and t-butyl (meth)acrylate. Examples of the alkyl (meth)acrylate having an alkyl group having 5 to 8 carbon atoms include pentyl (meth)acrylate, isopentyl (meth)acrylate, hexyl (meth)acrylate, 2-ethylhexyl (meth)acrylate, and cyclohexyl (meth)acrylate. These can be used alone or in combination of two or more.

Furthermore, examples of the copolymerizable vinyl monomer include an aromatic ring-containing vinyl monomer. Examples of the aromatic ring-containing vinyl monomer include an aromatic ring-containing (meth)acrylate. The aromatic ring-containing (meth)acrylate contains an aromatic ring. Examples of the aromatic ring include a benzene ring, a naphthalene ring, an anthracene ring, and a phenanthrene ring. More specifically, examples of the aromatic ring-containing (meth)acrylate include benzyl (meth)acrylate, phenoxyethyl (meth)acrylate, m-phenoxybenzyl (meth)acrylate, and 1-naphthylmethyl (meth)acrylate. These can be used alone or in combination of two or more. As the aromatic ring-containing (meth)acrylate, preferably, m-phenoxybenzyl (meth)acrylate and naphthylmethyl (meth)acrylate are used, more preferably, m-phenoxybenzyl acrylate and naphthylmethyl acrylate are used. Furthermore, in addition to the above, examples of the aromatic ring-containing vinyl monomer include styrene, α-methylstyrene, vinyl toluene, vinyl biphenyl, and divinyl benzene. These can be used alone or in combination of two or more. As the aromatic ring-containing vinyl monomer, preferably, an aromatic ring-containing (meth)acrylate is used.

Furthermore, examples of the copolymerizable vinyl monomer include a functional group (other than a hydroxyl group)-containing vinyl monomer. Examples of the functional group (other than a hydroxyl group)-containing vinyl monomer include a carboxy group-containing vinyl monomer, a glycidyl group-containing vinyl monomer, an amino group-containing vinyl monomer, a cyano group-containing vinyl monomer, an acetoacetoxy group-containing vinyl monomer, a sulfonic acid group-containing vinyl monomer, and a phosphoric acid group-containing vinyl monomer. Examples of the carboxy group-containing vinyl monomer include an acrylic acid. Examples of the glycidyl group-containing vinyl monomer include (meth)acrylate glycidyl. Examples of the amino group-containing vinyl monomer include (meth)acrylate 2-aminoethyl. Examples of the cyano group-containing vinyl monomer include (meth)acrylonitrile. Examples of the acetoacetoxy group-containing vinyl monomer include (meth)acrylate acetoacetoxy ethyl. Examples of the sulfonic acid group-containing vinyl monomer include allylsulfonic acid and a salt thereof. Examples of the phosphoric acid group-containing vinyl monomer include 2-methacroyloxyethyl acid phosphate. These can be used alone or in combination of two or more. As the functional group (other than a hydroxyl group)-containing vinyl monomer, preferably, a carboxy group-containing vinyl monomer is used, more preferably, an acrylic acid is used.

In addition to the above, examples of the copolymerizable vinyl monomer include the vinyl esters, an N-substituted unsaturated carboxylic acid amide, a heterocyclic vinyl compound, a halogenated vinylidene compound, α-olefins, dienes, and a cross-linkable vinyl monomer. Examples of the cross-linkable vinyl monomer include a polyfunctional (meth)acrylate, and more specifically include difunctional (meth)acrylate, trifunctional (meth)acrylate, and tetrafunctional or more (meth)acrylate. Examples of the difunctional (meth)acrylate include ethylene glycol di(meth)acrylate, butylene glycol di(meth)acrylate, hexanediol di(meth)acrylate, and oligoethylene glycol di(meth)acrylate. Examples of the trifunctional (meth)acrylate include trimethylol propane tri(meth)acrylate. Examples of the tetrafunctional or more(meth)acrylate include pentaerythritol tetra(meth)acrylate. These can be used alone or in combination of two or more.

These copolymerizable vinyl monomers can be used alone or in combination of two or more.

As the copolymerizable vinyl monomer, preferably, an aromatic ring-containing vinyl monomer is used. When the copolymerizable vinyl monomer contains an aromatic ring-containing vinyl monomer, the acrylic polyol contains an aromatic ring. In other words, the acrylic polyol is preferably an aromatic ring-containing acrylic polyol. According to the aromatic ring-containing acrylic polyol, a cured product (described below) having an excellent refractive index and excellent flexibility can be produced.

Furthermore, as the copolymerizable vinyl monomer, preferably, a carboxy group-containing vinyl monomer is used. When the carboxy group-containing vinyl monomer is contained, the acrylic polyol contains a carboxy group. In other words, the acrylic polyol is preferably a carboxy group-containing acrylic polyol. According to the carboxy group-containing acrylic polyol, a cured product (described below) having an excellent refractive index and excellent flexibility can be produced.

The mixing ratio of the hydroxyl group-containing (meth)acrylate to the copolymerizable vinyl monomer is appropriately set depending on the purpose and use. For example, with respect to 100 parts by mass of the total amount of the hydroxyl group-containing (meth)acrylate and the copolymerizable vinyl monomer, the hydroxyl group-containing (meth)acrylate is, for example, 1 part by mass or more, preferably 3 parts by mass or more. Furthermore, with respect to 100 parts by mass of the total amount of the hydroxyl group-containing (meth)acrylate and the copolymerizable vinyl monomer, the hydroxyl group-containing (meth)acrylate is 30 parts by mass or less, preferably 10 parts by mass or less.

Furthermore, with respect to 100 parts by mass of the total amount of the hydroxyl group-containing (meth)acrylate and the copolymerizable vinyl monomer, the copolymerizable vinyl monomer (total amount) is, for example, 70 parts by mass or more, preferably 90 parts by mass or more. Furthermore, with respect to 100 parts by mass of the total amount of the hydroxyl group-containing (meth)acrylate and the copolymerizable vinyl monomer, the copolymerizable vinyl monomer (total amount) is, for example, 99 parts by mass or less, preferably 97 parts by mass or less.

More specifically, when the aromatic ring-containing vinyl monomer is used, the aromatic ring-containing vinyl monomer is, for example, 70 parts by mass or more, preferably 90 parts by mass or more with respect to 100 parts by mass of the total amount of the hydroxyl group-containing (meth)acrylate and the copolymerizable vinyl monomer. Furthermore, the aromatic ring-containing vinyl monomer is, for example, 99 parts by mass or less, preferably 97 parts by mass or less with respect to 100 parts by mass of the total amount of the hydroxyl group-containing (meth)acrylate and the copolymerizable vinyl monomer.

Furthermore, when the carboxy group-containing vinyl monomer is used, the content ratio of the carboxy group-containing vinyl monomer is appropriately set so that the sum of the acid value and hydroxyl group value of the acrylic polyol (carboxy group-containing acrylic polyol) is in a range described below.

For example, with respect to 100 parts by mass of the total amount of the hydroxyl group-containing (meth)acrylate and the copolymerizable vinyl monomer, the carboxy group-containing vinyl monomer is, for example, 1 part by mass or more, preferably 5 parts by mass or more. Furthermore, with respect to 100 parts by mass of the total amount of the hydroxyl group-containing (meth)acrylate and the copolymerizable vinyl monomer, the carboxy group-containing vinyl monomer is, for example, 99 parts by mass or less, preferably 97 parts by mass or less.

The method of producing the acrylic polyol is not especially limited. For example, the above-described acrylic material component is copolymerized under a known organic solvent. Examples of the organic solvent include toluene. The mixing ratio of the organic solvent and the timing of blending thereof are appropriately set depending on the purpose and use.

**In** the production of the acrylic polyol, as necessary, a known polymerization initiator is used. Examples of the polymerization initiator include a known radical polymerization initiator (described below), more preferably, an azo compound (described below) and a peroxide (described below) are used, even more preferably, a peroxide (described below) is used, particularly preferably, t-butyl peroxy-2-ethylhexanoate is used. The mixing ratio of the polymerization initiator and the timing of blending thereof are appropriately set depending on the purpose and use.

The conditions for the polymerization of the acrylic material component are appropriately set depending on the purpose and use. For example, the polymerization temperature is, for example, 50°C or more, preferably 70°C or more. Furthermore, the polymerization temperature is, for example, 150°C or less, preferably 130°C or less. Furthermore, the polymerization time is, for example, 30 minutes or more, preferably 1 hour or more. Furthermore, the polymerization time is, for example, 12 hours or less, preferably 6 hours or less.

In this manner, an acrylic polyol is produced. For example, when an organic solvent is used in the above-described polymerization method, a solution and/or dispersion of the acrylic polyol is produced as the reaction mixture.

The solid content concentration of the solution and/or dispersion of the acrylic polyol is adjusted by a known method. For example, as necessary, the organic solvent can be added to the above-described reaction mixture. Furthermore, a part of the organic solvent can be removed from the above-described reaction mixture.

The solution and/or dispersion of the acrylic polyol has a solid content concentration of, for example, 30% by mass or more, preferably 40% by mass or more. Furthermore, the solution and/or dispersion of the acrylic polyol has a solid content concentration of, for example, 60% by mass or less, preferably 50% by mass or less.

The acrylic polyol has a number average molecular weight of, for example, more than 400, preferably 500 or more, more preferably 1000 or more. Furthermore, the acrylic polyol has a number average molecular weight is, for example, 20000 or less, preferably 15000 or less, more preferably 10000 or less, even more preferably 5000 or less.

The acrylic polyol has a hydroxyl group value of, for example, 1 mgKOH/g or more, preferably 3 mgKOH/g or more. Furthermore, the acrylic polyol has a hydroxyl group value of, for example, 500 mgKOH/g or less, preferably 300 mgKOH/g or less. The hydroxyl group value is measured in conformity with the description in JIS K 1557-1 (2007) (hereinafter, the same applies).

Furthermore, when the acrylic polyol does not contain a carboxy group-containing acrylic polyol, the acrylic polyol has a hydroxyl group value of, preferably 5 mgKOH/g or more, more preferably 10 mgKOH/g or more, even more preferably 15 mgKOH/g or more, particularly preferably 20 mgKOH/g or more. Furthermore, the acrylic polyol has a hydroxyl group value of, preferably 200 mgKOH/g or less, even more preferably 100 mgKOH/g or less, particularly preferably 50 mgKOH/g or less.

Furthermore, when the acrylic polyol contains a carboxy group-containing acrylic polyol, the sum of the acid value and hydroxyl group value of the acrylic polyol (carboxy group-containing acrylic polyol) is preferably 1 mgKOH/g or more, more preferably 3 mgKOH/g or more. Furthermore, the sum of the acid value and hydroxyl group value of the acrylic polyol (carboxy group-containing acrylic polyol) is, preferably 20 mgKOH/g or less, more preferably 10 mgKOH/g or less. The sum of the acid value and hydroxyl group value is measured in conformity with the description in JIS K 1557-5 (2007) (hereinafter, the same applies).

Furthermore, when the acrylic polyol contains a carboxy group-containing acrylic polyol, the acid value of the acrylic polyol (carboxy group-containing acrylic polyol) is, for example, 1 mgKOH/g or more, preferably 3 mgKOH/g or more. Furthermore, the acid value of the acrylic polyol (carboxy group-containing acrylic polyol) is, for example, 20 mgKOH/g or less, preferably 10 mgKOH/g or less. The acid value is measured in conformity with the description in JIS K 1557-5 (2007) (hereinafter, the same applies).

In addition to the above-described macropolyol, the polyol can contain a low molecular weight polyol. The low molecular weight polyol has two or more hydroxyl groups in one molecule. The low molecular weight polyol is an organic compound having a relatively low molecular weight. The low molecular weight polyol has a molecular weight of, for example, 40 or more, for example, 400 or less.

Examples of the low molecular weight polyol include a dihydric alcohol, a trihydric alcohol, and a tetrahydric or more alcohol. Examples of the dihydric alcohol include ethylene glycol, 1,2-propane diol, 1,3-propane diol, 1,2-butane diol, 1,3-butane diol, 1,4-butane diol, 1,5-pentane diol, 1,6-hexane diol, neopentyl glycol, diethylene glycol, triethylene glycol, and dipropylene glycol. Examples of the trihydric alcohol include glycerin and trimethylol propane. Examples of the tetrahydric or more alcohol include pentaerythritol and diglycerin. Furthermore, examples of the low molecular weight polyol also include a polymer obtained by adding and polymerizing alkylene (C2 to 3) oxide with a dihydric to tetrahydric alcohol so that the number average molecular weight is set to less than 400. These can be used alone or in combination of two or more. As the low molecular weight polyol, preferably, a dihydric alcohol and a trihydric alcohol are used, more preferably, a dihydric alcohol is used.

These polyols can be used alone or in combination of two or more. As the polyol, preferably, the macropolyol is used alone, more preferably, the acrylic polyol is used alone.

### (3-4) Embodiments of Polyurethane Resin Material

Examples of an embodiment of the polyurethane resin material include a one-component curable polyurethane resin and a two-component curable polyurethane resin. As an embodiment of the polyurethane resin material, preferably, a two-component curable polyurethane resin is used. The two-component curable polyurethane resin includes a curing agent and a main agent separately.

In the two-component curable polyurethane resin, the curing agent contains, for example, the above-described polyisocyanate. In the two-component curable polyurethane resin, the main agent contains, for example, the above-described polyol. The curing agent and the main agent are mixed together when being used to form a urethane cured product (cured resin described below). In the main agent, the polyol is may be diluted by a known organic solvent. Furthermore, in the curing agent, the polyisocyanate may be diluted with a known organic solvent.

The two-component curable polyurethane resin preferably includes a main agent containing the above-described acrylic polyol and a curing agent containing the above-described polyisocyanate. In this manner, a cured product (described below) having an excellent refractive index and excellent flexibility can be produced.

The polyisocyanate and the polyol are subjected to a urethane-formation reaction by a known method to produce a cured polyurethane resin. In the urethane-formation reaction, the mixing ratio of the polyisocyanate to the polyol is adjusted, for example, based on the equivalent ratio (OH/NCO) of the hydroxyl groups in the polyol with respect to the isocyanate groups in the polyisocyanate. With respect to the isocyanate groups in the polyisocyanate, the equivalent ratio (OH/NCO) of the hydroxyl groups in the polyol is, for example, 0.5 or more, preferably 0.8 or more, more preferably 0.95 or more. Furthermore, with respect to the isocyanate groups in the polyisocyanate, the equivalent ratio (OH/NCO) of the hydroxyl groups in the polyol is, for example, 2.0 or less, preferably 1.5 or less, more preferably 1.1 or less.

Furthermore, when the polyol contains the above-described carboxy group-containing acrylic polyol, the mixing ratio of the polyisocyanate to the polyol is adjusted, for example, based on the equivalent ratio (OH+COOH/NCO) of (the total amount of) the hydroxyl groups and the carboxy groups in the polyol with respect to the isocyanate groups in the polyisocyanate. With respect to the isocyanate groups in the polyisocyanate, the equivalent ratio (OH+COOH/NCO) of (the total amount of) the hydroxyl groups and the carboxy groups in the polyol is, for example, 0.5 or more, preferably 0.8 or more, more preferably 0.95 or more. Furthermore, with respect to the isocyanate groups in the polyisocyanate, the equivalent ratio (OH+COOH/NCO) of (the total amount of) the hydroxyl groups and the carboxy groups in the polyol is, for example, 2.0 or less, preferably 1.5 or less, more preferably 1.1 or less.

### (3-5) Acrylic Resin Material

The acrylic resin material is a curable compound for producing a cured acrylic resin (described below). The acrylic resin material contains, for example, a monomer component and a polymerization initiator.

### (3-6) Monomer Component

The monomer component contains, for example, a radically polymerizable monomer. The radically polymerizable monomer is a monomer that can produce a cured acrylic resin by a radical polymerization.

Examples of the radically polymerizable monomer include the monomers described above as the material of the acrylic polyol. More specifically, examples of the radically polymerizable monomer include the above-described alkyl (meth)acrylate, the above-described hydroxyl group-containing (meth)acrylate, the above-described aromatic ring-containing vinyl monomer, the above-described carboxy group-containing vinyl monomer, the above-described glycidyl group-containing vinyl monomer, the above-described amino group-containing vinyl monomer, the above-described cyano group-containing vinyl monomer, the above-described acetoacetoxy group-containing vinyl monomer, the above-described sulfonic acid group-containing vinyl monomer, the above-described phosphoric acid group-containing vinyl monomer, the above-described vinyl esters, the above-described N-substituted unsaturated carboxylic acid amide, the above-described heterocyclic vinyl compound, the above-described halogenated vinylidene compound, the above-described α-olefins, the above-described dienes, and the above-described cross-linkable vinyl monomer. These can be used alone or in combination of two or more.

As the radically polymerizable monomer, preferably, a cross-linkable vinyl monomer is used, more preferably, a polyfunctional (meth)acrylate is used, even more preferably, a trifunctional (meth)acrylate is used, particularly preferably a trimethylol propane tri(meth)acrylate is used.

In other words, the monomer component preferably contains a cross-linkable vinyl monomer, even more preferably contains a polyfunctional (meth)acrylate, even more preferably contains a trifunctional (meth)acrylate, particularly preferably contains a trimethylol propane tri(meth)acrylate.

Furthermore, as the radically polymerizable monomer, preferably, a radically polymerizable monomer other than the cross-linkable vinyl monomer (hereinafter the other monomer) is also used.

In other words, the monomer component preferably contains a cross-linkable vinyl monomer and the other monomer. As the other monomer, preferably, the above-described alkyl (meth)acrylate and/or the above-described aromatic ring-containing vinyl monomer are/is used.

The content ratios of the cross-linkable vinyl monomer and the other radically polymerizable monomer are appropriately set depending on the purpose and use.

For example, with respect to the total amount of the cross-linkable vinyl monomer and the other radically polymerizable monomer, the content ratio of the cross-linkable vinyl monomer is, for example, 10% by mass or more, preferably 20% by mass or more, more preferably 40% by mass or more, even more preferably 50% by mass or more, particularly preferably 60% by mass or more. Furthermore, with respect to the total amount of the cross-linkable vinyl monomer and the other radically polymerizable monomer, the content ratio of the cross-linkable vinyl monomer is, for example, 99% by mass or less, preferably 90% by mass or less, more preferably 80% by mass or less, even more preferably 70% by mass or less, particularly preferably 65% by mass or less.

For example, with respect to the total amount of the cross-linkable vinyl monomer and the other radically polymerizable monomer, the content ratio of the other radically polymerizable monomer is, for example, 1% by mass or more, preferably 10% by mass or more, more preferably 20% by mass or more, even more preferably 30% by mass or more, particularly preferably 35% by mass or more. Furthermore, with respect to the total amount of the cross-linkable vinyl monomer and the other radically polymerizable monomer, the content ratio of the other radically polymerizable monomer is, for example, 90% by mass or less, preferably 80% by mass or less, more preferably 60% by mass or less, even more preferably 50% by mass or less, particularly preferably 40% by mass or less.

### (3-7) Polymerization Initiator

Examples of the polymerization initiator include a known radical polymerization initiator. Examples of the radical polymerization initiator include an active-energy-ray radical polymerization initiator and a thermal radical polymerization initiator.

Examples of the active-energy-ray radical polymerization initiator include a photo-radical polymerization initiator. Examples of the photo-radical polymerization initiator include an alkyl phenone-based photo-polymerization initiator, an acyl phosphine oxide-based photo-polymerization initiator, an oxime ester-based photo-polymerization initiator, a carbazole phenone-based photo-polymerization initiator, an acridine-based photo-polymerization initiator, a triazine-based photo-polymerization initiator, and a benzoyl-based photo-polymerization initiator. These can be used alone or in combination of two or more.

Examples of the thermal radical polymerization initiator include an azo compound and a peroxide. Examples of the azo compound include azoisobutyronitrile and dimethylazoisobutyrate. Examples of the peroxide include benzoyl peroxide, ketone peroxide, peroxyketal, hydro peroxide, dialkyl peroxide, diacyl peroxide, peroxyester, t-butyl peroxy-2-ethylhexanoate, and peroxydicarbonate. These can be used alone or in combination of two or more.

The active-energy-ray radical polymerization initiator may be able to function as a thermal radical polymerization initiator. Furthermore, the thermal radical polymerization initiator may be able to function as an active-energy-ray radical polymerization initiator.

Examples of the radical polymerization initiator include a commercially available product. Examples of the commercially available radical polymerization initiator include the Omnirad series manufactured by IGM Resins B.V. and the PERCURE series manufactured by NOF CORPORATION. These can be used alone or in combination of two or more.

As the polymerization initiator, preferably, an active-energy-ray radical polymerization initiator is used, more preferably, a photo-polymerization initiator is used. The mixing ratio of the polymerization initiator and the timing of blending thereof are appropriately set depending on the purpose and use.

### (3-8) Blending Formulation of Acrylic Resin Material

Examples of an embodiment of the acrylic resin material include a mixture containing the above-described radically polymerizable monomer and the above-described polymerization initiator. The acrylic resin material may contain a known organic solvent. Preferably, the acrylic resin material does not contain an organic solvent and consists of the above-described radically polymerizable monomer and the above-described polymerization initiator.

### (4) Embodiment of Curable Composition

As described above, the curable composition contains, for example, a plasticizer and a curable compound. Preferably, the curable composition contains a plasticizer, and a polyurethane resin material and/or an acrylic resin material as a curable compound. More preferably, the curable composition contains a plasticizer and a polyurethane resin material, or contains a plasticizer and an acrylic resin material.

The mixing ratio of the plasticizer to the curable compound is not especially limited, and is appropriately set depending on the purpose and use. For example, with respect to 100 parts by mass of the solid content of the curable compound, the plasticizer is, for example, 1 part by mass (phr) or more, preferably 5 parts by mass (phr) or more. Furthermore, with respect to 100 parts by mass of the solid content of the curable compound, the plasticizer is, for example, 70 parts by mass (phr) or less, preferably 50 parts by mass (phr) or less.

Furthermore, for example, with respect to the total amount (based on the solid content) of the plasticizer and the curable compound, the plasticizer is, for example, 1% by mass or more, preferably 3% by mass or more. Furthermore, with respect to the total amount (based on the solid content) of the plasticizer and the curable compound, the plasticizer is, for example, 50% by mass or less, preferably 35% by mass or less.

Furthermore, for example, with respect to the total amount (based on the solid content) of the plasticizer and the curable compound, the curable compound is, for example, 50% by mass or more, preferably 65% by mass or more. Furthermore, with respect to the total amount (based on the solid content) of the plasticizer and the curable compound, the curable compound is, for example, 99% by mass or less, preferably 97% by mass or less.

Furthermore, the embodiment of containing the plasticizer is not especially limited. For example, when the curable compound is a two-component curable polyurethane resin, the plasticizer may be added to the curing agent (polyisocyanate) of the two-component curable polyurethane resin. Furthermore, the plasticizer may be added to the main agent (polyol) of the two-component curable polyurethane resin. Furthermore, the plasticizer may be added to both of the main agent and curing agent of the two-component curable polyurethane resin.

Furthermore, the plasticizer may be prepared separately from the main agent and curing agent of the two-component curable polyurethane resin. When the plasticizer is separately prepared, the plasticizer may be blended at the same time as the mixing of the main agent and the curing agent, or may be blended into the mixture of the main agent and the curing agent after the mixing thereof.

Furthermore, for example, when the curable compound is an acrylic resin material, the plasticizer may be mixed with the radically polymerizable monomer in advance. Furthermore, the plasticizer may be mixed with the polymerization initiator in advance. Furthermore, the plasticizer may be blende at the same time as the mixing of the radically polymerizable monomer and the polymerization initiator, or may be blended into the mixture of the radically polymerizable monomer and the polymerization initiator after the mixing thereof.

### (5) Secondary Component

As necessary, the curable composition can contain an additive as a secondary component. The secondary component is a component contained in a content ratio of a predetermined value or less with respect to the whole. The content ratio of the secondary component with respect to the whole is, for example, 10% by mass or less. **In** other words, the ratio of the additive is 10% by mass or less with respect to the total amount of the solid content of the curable composition.

Examples of the additive include an ultraviolet absorber (ultraviolet absorber), a light-resistant stabilizer (light stabilizer), and an antioxidant. Furthermore, examples of the additive include a heat-resistant stabilizer, a cross-linkage agent, a silane coupling agent, a defoaming agent, a leveling agent, a fungicide, a corrosion inhibitor, a flatting agent, a flame retardant, a thixotropic agent, a tackifier, a thickener, a lubricant, an anti-static agent, a surfactant, a reaction retarder, a dehydrating agent, a dye, an inorganic pigment, an organic pigment, an anti-tack agent, an inorganic filler, and an organic filler. These can be used alone or in combination of two or more. The adding amount of the additive and the timing of adding thereof are appropriately set depending on the purpose and use.

In view of the weather resistance of the cured product (described below), as the additive, preferably, an ultraviolet absorber (UV absorber), a light-resistant stabilizer (light stabilizer), and an antioxidant are used. In other words, the curable composition preferably contains at least one selected from the group consisting of an ultraviolet absorber, a light-resistant stabilizer, and an antioxidant. In view of the weather resistance of the cured product (described below), the ratio of the additive (at least one additive selected from the group consisting of an ultraviolet absorber, a light-resistant stabilizer, and an antioxidant) with respected to 1 part by mass of (the total amount of) the above-described curable composition is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the ratio of the additive (at least one additive selected from the group consisting of an ultraviolet absorber, a light-resistant stabilizer, and an antioxidant) with respected to 1 part by mass of (the total amount of) the above-described curable composition is, for example, 100000×10⁻⁶ parts by mass (ppm) or less, preferably 10000×10⁻⁶ parts by mass (ppm) or less, more preferably 1000×10⁻⁶ parts by mass (ppm) or less.

The ultraviolet absorber is not especially limited, and examples thereof include a known ultraviolet absorber (for example, shown in a catalog published by ADEKA and a catalog published by CLARIAN). More specifically, examples of the ultraviolet absorber include a benzotriazole-based ultraviolet absorber, a benzophenone-based ultraviolet absorber, a benzylidene-based ultraviolet absorber, and a triazine-based ultraviolet absorber. These can be used alone or in combination of two or more. In view of the weather resistance of the cured product (described below), as the ultraviolet absorber, preferably, a benzotriazole-based ultraviolet absorber is used. The mixing ratio of the ultraviolet absorber with respect to 1 part by mass of the above-described curable composition is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the ultraviolet absorber with respect to 1 part by mass of the above-described curable composition is, for example, 100000×10⁻⁶ parts by mass (ppm) or less, preferably 10000×10⁻⁶ parts by mass (ppm) or less, more preferably 1000×10⁻⁶ parts by mass (ppm) or less.

The light-resistant stabilizer is not especially limited, and examples thereof include a known light-resistant stabilizer (for example, shown in a catalog published by ADEKA). More specifically, examples of the light-resistant stabilizer include a hindered amine-based (HALS) light-resistant stabilizer and a benzoate-based light-resistant stabilizer. These can be used alone or in combination of two or more. In view of the weather resistance of the cured product (described below), as the light-resistant stabilizer, preferably, a hindered amine-based (HALS) light-resistant stabilizer is used. The mixing ratio of the light-resistant stabilizer with respect to 1 part by mass of the above-described curable composition is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the light-resistant stabilizer with respect to 1 part by mass of the above-described curable composition is, for example, 100000×10⁻⁶ parts by mass (ppm) or less, preferably 10000×10⁻⁶ parts by mass (ppm) or less, more preferably 1000×10⁻⁶ parts by mass (ppm) or less.

The antioxidant is not especially limited, and examples thereof include a known antioxidant (for example, shown in a catalog published by ADEKA). More specifically, examples of the antioxidant include a phenol-based antioxidant, a phosphorus-based antioxidant, and a thiophene-based antioxidant. These can be used alone or in combination of two or more. In view of the weather resistance of the cured product (described below), as the antioxidant, preferably, a phenol-based antioxidant. The mixing ratio of the antioxidant with respect to 1 part by mass of the above-described curable composition is, for example, 1 ×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the antioxidant with respect to 1 part by mass of the above-described curable composition is, for example, 100000×10⁻⁶ parts by mass (ppm) or less, preferably 10000×10⁻⁶ parts by mass (ppm) or less, more preferably 1000×10⁻⁶ parts by mass (ppm) or less.

### (6) Uses

The above-described curable composition is preferably used in various industrial fields. Examples of the use of the curable composition include a coating agent, a paint, and an adhesive. The use of the curable composition is preferably an adhesive.

The adhesive is an uncured curable composition, and by curing it, a cured product of an adhesive (a cured product described below) is formed. The cured product of an adhesive adheres an adherent.

Then, according to the above-described curable composition, a cured product having an excellent refractive index and excellent flexibility can be produced. Therefore, the above-described curable composition is preferably used as an optical curable composition. Examples of the use of the optical curable composition include an optical coating agent, an optical paint, and an optical adhesive.

### 4. Cured Product

### (1) Main Component

The cured product contains the above-described plasticizer and the cured resin as a main component. The main component is a component contained in a content ratio of a predetermined value or more with respect to the whole. The content ratio of the main component with respect to the whole is, for example, 90% by mass or more. The total amount of the solid content of the plasticizer and the cured resin with respect to the total amount of the solid content of the cured product is 90% by mass or more.

The cured product is formed by curing the above-described curable composition in a known method. The method of curing the curable composition is appropriately selected depending on the type of the curable compound.

### (2) Cured Resin

The cured resin contains a cured product of the above-described curable compound, and preferably consists of a cured product of the above-described curable compound.

More specifically, examples of the cured resin include a cured polyurethane resin, a cured polyolefin resin, a cured polyamine resin, a cured amide resin, a cured urea resin, a cured phenol resin, a cured epoxy resin, a cured acrylic resin, a cured melamine resin, and a cured alkyd resin. These can be used alone or in combination of two or more.

As the cured resin, preferably, a cured polyurethane resin and a cured acrylic resin are used.

Examples of the cured polyurethane resin include a cured product of the above-described polyurethane resin material. More specifically, examples of the cured polyurethane resin include a reaction product of a main agent containing the above-described polyol and a curing agent containing the above-described polyisocyanate. By such a cured resin, a cured product having a more excellent refractive index and more excellent flexibility can be produced. More specifically, when the cured resin contains the cured polyurethane resin (a reaction product of a main agent containing the above-described acrylic polyol and a curing agent containing the above-described polyisocyanate), a cured product satisfying the physical properties (refractive index and flexibility) described below can more easily be produced.

Examples of the cured acrylic resin include a cured product of the above-described acrylic resin material. More specifically, examples of the cured acrylic resin include a reaction product obtained by the radical polymerization reaction of the above-described radically polymerizable monomer. The method of producing the cured acrylic resin is not especially limited. For example, when an active-energy-ray radical polymerization initiator is used, the acrylic resin material is irradiated with an active energy ray having a predetermined wavelength. Furthermore, when a thermal radical polymerization initiator is used, the acrylic resin material is heated to a predetermined temperature. In this manner, the polymerization initiator is activated, and the radically polymerizable monomer is subjected to a radical polymerization reaction, thereby producing a cured acrylic resin. In particular, when the cured resin contains a cured acrylic resin (a radically polymerized product of the above-described monomer component), a cured product having excellent flexibility and a particularly excellent refractive index is produced.

### (3) Secondary Component

As necessary, the cured product can contain the above-described additive as a secondary component. The secondary component is a component contained in a content ratio of a predetermined value or less with respect to the whole. The content ratio of the secondary component with respect to the whole is, for example, 10% by mass or less. In other words, the ratio of the additive with respect to the total amount of the solid content of the cured product is 10% by mass or less.

In view of the weather resistance of the cured product, as the additive, preferably, an ultraviolet absorber (UV absorber), a light-resistant stabilizer (light stabilizer), and an antioxidant are used. In other words, the cured product preferably contains at least one selected from the group consisting of an ultraviolet absorber, a light-resistant stabilizer, and an antioxidant. In view of the weather resistance of the cured product, the ratio of the additive (at least one additive selected from the group consisting of an ultraviolet absorber, a light-resistant stabilizer, and an antioxidant) with respect to 1 part by mass (of the total amount) of the above-described cured product is, for example, 1 × 10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the ratio of the additive (at least one additive selected from the group consisting of an ultraviolet absorber, a light-resistant stabilizer, and an antioxidant) with respect to 1 part by mass (of the total amount) of the above-described cured product is, for example, 100000×10⁻⁶ parts by mass (ppm) or less, preferably 10000×10⁻⁶ parts by mass (ppm) or less, more preferably 1000×10⁻⁶ parts by mass (ppm) or less.

The ultraviolet absorber is not especially limited, and examples thereof include a known ultraviolet absorber (for example, shown in a catalog published by ADEKA). More specifically, examples of the ultraviolet absorber include a benzotriazole-based ultraviolet absorber, a benzophenone-based ultraviolet absorber, a benzylidene-based ultraviolet absorber, and a triazine-based ultraviolet absorber. These can be used alone or in combination of two or more. In view of the weather resistance of the cured product, as the ultraviolet absorber, preferably, a benzotriazole-based ultraviolet absorber is used. The mixing ratio of the ultraviolet absorber with respect to 1 part by mass of the above-described cured product is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the ultraviolet absorber with respect to 1 part by mass of the above-described cured product is, for example, 100000×10⁻⁶ parts by mass (ppm) or less, preferably 10000×10⁻⁶ parts by mass (ppm) or less, more preferably 1000×10⁻⁶ parts by mass (ppm) or less.

The light-resistant stabilizer (light stabilizer) is not especially limited, and examples thereof include a known light-resistant stabilizer (for example, shown in a catalog published by ADEKA). More specifically, examples of the light-resistant stabilizer include a hindered amine-based (HALS) light-resistant stabilizer and a benzoate-based light-resistant stabilizer. These can be used alone or in combination of two or more. In view of the weather resistance of the cured product, as the light-resistant stabilizer, preferably, a hindered amine-based (HALS) light-resistant stabilizer is used. The mixing ratio of the light-resistant stabilizer with respect to 1 part by mass of the above-described cured product is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the light-resistant stabilizer with respect to 1 part by mass of the above-described cured product is, for example, 100000×10⁻⁶ parts by mass (ppm) or less, preferably 10000×10⁻⁶ parts by mass (ppm) or less, more preferably 1000×10⁻⁶ parts by mass (ppm) or less.

The antioxidant is not especially limited, and examples thereof include a known antioxidant (for example, shown in a catalog published by ADEKA). More specifically, examples of the antioxidant include a phenol-based antioxidant, a phosphorus-based antioxidant, and a thiophene-based antioxidant. These can be used alone or in combination of two or more. In view of the weather resistance of the cured product, as the antioxidant, preferably, a phenol-based antioxidant is used. The mixing ratio of the antioxidant with respect to 1 part by mass of the above-described cured product is, for example, 1×10⁻⁶ parts by mass (ppm) or more, preferably 10×10⁻⁶ parts by mass (ppm) or more, more preferably 100×10⁻⁶ parts by mass (ppm) or more. Furthermore, in view of the low cost, the mixing ratio of the antioxidant with respect to 1 part by mass of the above-described cured product is, for example, 100000×10⁻⁶ parts by mass (ppm) or less, preferably 10000×10⁻⁶ parts by mass (ppm) or less, more preferably 1000×10⁻⁶ parts by mass (ppm) or less.

### (4) Physical Properties

The above-described cured product includes the above-described compound, and thus has both an excellent refractive index and excellent flexibility.

The refractive index of the cured product is relatively high. The cured product has a refractive index of, for example, 1.45 or more, preferably 1.50 or more, more preferably 1.55 or more, even more preferably 1.60 or more, particularly preferably 1.61 or more. Furthermore, the cured product has a refractive index of, for example, 1.80 or less, preferably 1.70 or less. The refractive index is measured in conformity with Examples described below.

The tensile storage elastic modulus (E') of the cured product at 25°C is a relatively low. The cured product has a tensile storage elastic modulus (E') at 25°C of, for example, 1000 MPa or less, preferably 800 MPa or less, more preferably 500 MPa or less, even more preferably 300 MPa or less, even more preferably 200 MPa or less, even more preferably 100 MPa or less, particularly preferably 50 MPa or less. Furthermore, the cured product has a tensile storage elastic modulus (E') at 25°C of, for example, 1 MPa or more. The tensile storage elastic modulus (E') is measured in conformity with Examples described below.

### (5) Uses

The above-described cured product is preferably used in various industrial fields. Examples of the use of the cured product include a resin molded product, a film, and a pressure-sensitive adhesive. The use of the cured product is preferably a pressure-sensitive adhesive.

The pressure-sensitive adhesive is a cured curable composition. For example, the pressure-sensitive adhesive is a cured product of the curable composition containing a polyurethane resin material and/or an acrylic resin material (preferably, an acrylic resin material) as a curable compound. The pressure-sensitive adhesive has a relatively low glass transition temperature (0°C or less) and has adhesion (tackiness).

Then, the above-described cured product has an excellent refractive index and excellent flexibility. Therefore, the above-described cured product is preferably used as an optical resin. Examples of the use of the optical resin include an optical lens, an optical film, and an optical pressure-sensitive adhesive.

Then, the above-described curable composition and the adhesive and pressure-sensitive adhesive contain the above-described plasticizer. Therefore, according to such a curable composition, a cured product having both a refractive index and flexibility can be produced. Examples

Next, the present invention is described based on Examples and Comparative Examples. The present invention is, however, not limited to them. The "parts" and "%" are based on mass unless otherwise specified. The specific numeral values used in the description below, such as mixing ratios (content ratios), physical property values, and parameters, can be replaced with the corresponding mixing ratios (content ratios), physical property values, and parameters in the above-described "DESCRIPTION OF THE EMBODIMENTS", including the upper limit values (numeral values defined with "or less", and "less than") or the lower limit values (numeral values defined with "or more", and "more than").

### 1. Synthesis of Plasticizer

### Example 1 (Bn-GST)

4-benzylthiomethyl-1,8-bis benzylthio-3,6-dithiaoctane (Bn-GST) was synthesized by the method below.

First, 40.5 g (595.0 mmol) of sodium ethoxide and 300mL of ethanol were charged into a four-neck flask equipped with a stirrer, a thermometer, a nitrogen inlet line, and a dripping funnel, and stirred. In this manner, a solution of sodium ethoxide was produced.

Next, 50.0 g (191.9 mmol) of 4-mercaptomethyl-1,8-dimercapto-3,6-dithiaoctane (GST) was slowly charged into the solution of sodium ethoxide while being stirred. In this manner, a reaction solution of the sodium ethoxide and GST was produced.

Next, the reaction solution was cooled in an ice bath. Next, while the internal temperature of the reaction solution was maintained at 10°C or less, 101.8 (595.0 mmol) of benzyl bromide was added dropwise into the reaction solution.

With respect to the mercapto groups in the GST, the equivalent ratio (bromine atoms/mercapto groups) of the bromine atoms in the benzyl bromide was 1.03.

Next, the ice bath was detached from the reaction solution. In this manner, the temperature of the reaction solution was allowed to come to room temperature. Furthermore, the reaction solution was stirred for one night.

Thereafter, 300mL of water was further added to the reaction solution. Furthermore, dilute hydrochloric acid was added to the reaction solution. In this manner, the pH of the reaction solution was adjusted to 6.0 or less. The pH was determined using pH test paper.

Thereafter, 500mL of dichloromethane was added to the reaction solution. Then, the mixture of them was separated to take an organic phase therefrom. Next, the organic phase was concentrated by an evaporator. In this manner, a concentrated product of the reaction solution was produced.

The concentrated product (crude product) was diluted with 100mL of dichloromethane, thereby producing a diluted solution. The diluted solution was passed through 100mL of silica gel. Furthermore, 300mL of dichloromethane was used to cause the diluted solution to flow out. Thereafter, the diluted solution was concentrated by an evaporator. In this manner, 4-benzylthiomethyl-1,8-bis benzylthio-3,6-dithiaoctane (Bn-GST) was produced as a reaction product.

The Bn-GST was a light yellowish transparent liquid. The amount of the collected Bn-GST was 84.2 g. Furthermore, the Bn-GST had a viscosity (25°C) of 500mPa·s. Furthermore, the Bn-GST had a refractive index (nD) of 1.63.

The Bn-GST was identified by ¹H-NMR (400MHz, CDCl₃) as described below.
¹H-NMR (400MHz, CDCl₃):
δ7.19-7.37(m, 15H)
δ3.70-3.78(m, 6H)
δ2.49-2.85(m, 13H)

### Example 2 (Bz-GST)

4-benzoylthiomethyl-1,8-bis benzoylthio-3,6-dithiaoctane (Bz-GST) was synthesized by the method below.

First, 30.0 g (115.2 mmol) of 4-mercaptomethyl-1,8-dimercapto-3,6-dithiaoctane (GST) was charged in a four-neck flask equipped with a stirrer, a thermometer, a nitrogen inlet line, and a dripping funnel.

Next, 100mL of dichloromethane was added to the above-described flask. In this manner, the solution of GST was produced.

Next, 38.5 g (380.0 mmol) of triethylamine was slowly charged into the solution of GST while being stirred. In this manner, a reaction solution of triethylamine and GST was produced.

Next, the reaction solution was cooled in an ice bath. Next, while the internal temperature of the reaction solution was maintained at 10°C or less, 53.4 g (380.0 mmol) of benzoyl chloride was added dropwise into the reaction solution.

With respect to the mercapto groups in the GST, the equivalent ratio (chlorine atoms/the mercapto groups) of chlorine atoms in the benzoyl chloride was 1.10.

Next, the ice bath was detached from the reaction solution. In this manner, the temperature of the reaction solution was allowed to come to room temperature. Furthermore, the reaction solution was stirred for one night.

Thereafter, 300mL of water and 200mL of dichloromethane were added to the reaction solution. Then, the mixture of them was separated to take an organic phase therefrom. Next, the organic phase was washed with dilute hydrochloric acid. The organic phase was further washed with a saturated sodium bicarbonate aqueous solution. Thereafter, the organic phase was concentrated by an evaporator. In this manner, a concentrated product of the reaction solution was produced.

The concentrated product (crude product) was diluted with 100mL of dichloromethane, thereby producing a diluted solution. The diluted solution was passed through 100mL of silica gel. Furthermore, 300mL of dichloromethane was used to cause the diluted solution to flow out. Thereafter, the diluted solution was concentrated by an evaporator. In this manner, 4-benzoylthiomethyl-1,8-bis benzoylthio-3,6-dithiaoctane (Bz-GST) was produced as a reaction product.

The Bz-GST was a colorless and transparent liquid. The amount of the collected Bz-GST was 63.2g. Furthermore, the Bz-GST had a viscosity (25°C) of 5000mPa·s. Furthermore, the Bz-GST had a refractive index (nD) of 1.65.

THe Bz-GST was identified by ¹H-NMR (400MHz, CDCl₃) as described below.
¹H-NMR (400MHz, CDCl₃):
δ7.89-8.01 (m, 6H)
δ7.51-7.61 (m, 3H)
δ7.37-7.48 (m, 6H)
δ2.79-3.59 (m, 13H)

### Example 3 (Ac-GST)

4- acetylthiomethyl-1,8-bis acetylthio-3,6-dithiaoctane (Ac-GST) was synthesized by the method below.

First, 5.00 g (19.2 mmol) of 4-mercaptomethyl-1,8-dimercapto-3,6-dithiaoctane (GST) was charged into a four-neck flask equipped with a stirrer, a thermometer, a nitrogen inlet line, and a dripping funnel.

Next, 20mL of dichloromethane was added to the above-described flask. In this manner, a solution of GST was produced.

Next, 6.41g (63.3mmol) of triethylamine was slowly charged into the solution of GST while being stirred. In this manner, a reaction solution of triethylamine and GST was produced.

Next, the reaction solution was cooled in an ice bath. Next, while the internal temperature of the reaction solution was maintained at 10°C or less, 6.47 (63.3 mmol) of acetic anhydride was added dropwise into the reaction solution.

With respect to the mercapto groups in the GST, the equivalent ratio (carboxy groups/mercapto groups) of the carboxy groups of the acetic anhydride was 2.20.

Next, the ice bath was detached from the reaction solution. In this manner, the temperature of the reaction solution was allowed to come to room temperature. Furthermore, the reaction solution was stirred for one night.

Thereafter, 30mL of water and 20mL of dichloromethane were added to the reaction solution. Then, the mixture of them was separated to take an organic phase therefrom. Next, the organic phase was washed with dilute hydrochloric acid.

The organic phase was further washed with a saturated sodium bicarbonate aqueous solution. Thereafter, the organic phase was concentrated by an evaporator. In this manner, a concentrated product of the reaction solution was produced.

The concentrated product (crude product) was diluted with 10mL of dichloromethane, thereby producing a diluted solution. The diluted solution was passed through 10mL of silica gel. Furthermore, 30mL of dichloromethane was used to cause the diluted solution to flow out. Thereafter, the diluted solution was concentrated by an evaporator. In this manner, 4-acetylthiomethyl-1,8-bis acetylthio-3,6-dithiaoctane (Ac-GST) was produced as a reaction product.

The Ac-GST was a colorless and transparent liquid. The amount of the collected Ac-GST was 7.21g. Furthermore, the Ac-GST had a viscosity (25°C) of 200mPa·s. Furthermore, the Ac-GST had a refractive index (nD) of 1.58.

The Ac-GST was identified by ¹H-NMR (400MHz, CDCl₃) as described below.
¹H-NMR (400MHz, CDCl₃):
δ2.71-3.34 (m, 13H)
δ2.33-2.38 (m, 9H)

### Example 4 (PA-GST)

4-phenyl acetylthiomethyl-1,8-bisphenyl acetylthio-3,6-dithiaoctane (PA-GST) was synthesized by the method below.

First, 50.0 g (191.9 mmol) of 4-mercaptomethyl-1,8-dimercapto-3,6-dithiaoctane (GST) was charged into a four-neck flask equipped with a stirrer, a thermometer, a nitrogen inlet line, and a dripping funnel.

Next, 250 g of toluene was added to the above-described flask. In this manner, a solution of GST was produced.

Next, 61.8 g (610.3 mmol) of triethylamine was slowly charged into the solution of GST while being stirred. In this manner, a reaction solution of triethylamine and GST was produced.

Next, the reaction solution was cooled in an ice bath. Next, while the internal temperature of the reaction solution was maintained at 15°C or less, 91.7 g (593.1 mmol) of phenyl acetyl chloride was added dropwise into the reaction solution.

With respect to the mercapto groups in the GST, the equivalent ratio (chlorine atoms/mercapto groups) of the chlorine atoms in the phenyl acetylchloride was1.03.

Next, after the completion of the addition by drops, while the internal temperature of the reaction solution was maintained at 15°C or less, the reaction solution was stirred for 2 hours.

Thereafter, 550 g of a 10% sodium chloride aqueous solution was added to the reaction solution. Then, the mixture of them was separated to take an organic phase therefrom. Next, the organic phase was washed with 500 mL of 1N dilute hydrochloric acid, and further washed with 500mL of a saturated sodium bicarbonate aqueous solution, and further washed with 500mL of purified water.

The washed solution was passed through an activated alumina (300 mesh, for chromatograph, basic). Furthermore, 120 g of toluene was used to cause the reaction product remaining in the alumina to flow out.

An anhydrous sodium sulfate was added to the passed-though washed solution, and stirred to remove the moisture remaining in the washed solution. The sodium sulfate was filtered with a pleated paper filter to produce a toluene diluted solution as a product. Thereafter, the diluted solution was concentrated by an evaporator. In this manner, 4-phenyl acetylthiomethyl-1,8-bisphenyl acetylthio-3,6-dithiaoctane (PA-GST) was produced as a reaction product.

The PA-GST was a light yellowish transparent liquid. The amount of the collected PA-GST was 105.2g. Furthermore, the PA-GST had a viscosity (25°C) of 1100mPa·s. Furthermore, the PA-GST had a refractive index (nD) of 1.62.

The PA-GST was identified by ¹H-NMR(400MHz, CDCl₃) as described below.
¹H-NMR (400MHz, CDCl₃):
δ7.21-7.37 (m, 15H)
δ3.80-3.84 (m, 6H)
δ2.64-3.25 (m, 13H)

### Example 5 (PP-GST)

4-(3-phenyl propionyl)thiomethyl-1,8-bis(3-phenyl propionyl)thio-3,6-dithiaoctane (PP-GST) was synthesized by the method below.

First, 50.0 g (191.9 mmol) of 4-mercaptomethyl-1,8-dimercapto-3,6-dithiaoctane (GST) was charged into a four-neck flask equipped with a stirrer, a thermometer, a nitrogen inlet line, and a dripping funnel.

Next, 250g of toluene was added to the above-described flask. In this manner, the solution of GST was produced.

Next, 61.8 g (610.3 mmol) of triethylamine was slowly charged into the solution of GST while being stirred. In this manner, a reaction solution of triethylamine and GST was produced.

Next, the reaction solution was cooled in an ice bath. Next, while the internal temperature of the reaction solution was maintained at 15°C or less, 100.0 g (593.1 mmol) of 3-phenyl propionyl chloride was added dropwise into the reaction solution.

With respect to the mercapto groups in the GST, the equivalent ratio (chlorine atoms/mercapto groups) of the chlorine atoms in the 3-phenyl propionyl chloride was 1.03.

Next, after the completion of the addition by drops, while the internal temperature of the reaction solution was maintained at 15°C or less, the reaction solution was stirred for 2 hours.

Thereafter, 550 g of a 10% sodium chloride aqueous solution was added to the reaction solution. Then, the mixture of them was separated to take an organic phase therefrom. Next, the organic phase was washed with 500mL of a 1N dilute hydrochloric acid, further washed with 500 mL of a saturated sodium bicarbonate aqueous solution, and further washed with 500mL of purified water.

The washed solution was passed through an activated alumina (300mesh, for chromatograph, basic). Furthermore, 120 g of toluene was used to cause the reaction product remaining in the alumina to flow out.

An anhydrous sodium sulfate was added to the passed-though washed solution, and stirred to remove the moisture remaining in the washed solution. The sodium sulfate was filtered with a pleated paper filter, thereby producing a toluene diluted solution as a product. Thereafter, the diluted solution was concentrated by an evaporator. In this manner, 4-(3-phenyl propionyl) thiomethyl-1,8-bis(3-phenyl propionyl)thio-3,6-dithiaoctane (PP-GST) was produced as a reaction product.

The PA-GST was a light yellowish transparent liquid. The amount of the collected PP-GST was 119.0 g. Furthermore, the PP-GST had a viscosity (25°C) of 400mPa·s. Furthermore, the PP-GST had a refractive index (nD) of 1.60.

The PP-GST was identified by ¹H-NMR (400MHz, CDCl₃) as described below.
¹H-NMR (400MHz, CDCl₃):
δ7.13-7.33 (m, 15H)
δ2.66-3.29 (m, 25H)

### Example 6 (Bz-FSH)

5,7-bis(benzoylmercaptomethyl)-1,11-bis(benzoylmercapto)-3,6,9-trithiaundecane(Bz-FSH) was synthesized by the method below.

First, 50.0 g (136.4 mmol) of 5,7-dimercaptomethyl-1,11-dimercapto-3,6,9-trithiaundecane (FSH) was charged into a four-neck flask equipped with a stirrer, a thermometer, a nitrogen inlet line, and a dripping funnel.

Next, 500g of toluene was added to the above-described flask. In this manner, a solution of FSH was produced.

Next, 42.8 g (422.7 mmol) of triethylamine was slowly charged into the solution of FSH while being stirred. In this manner, a reaction solution of the triethylamine and the FSH was produced.

Next, the reaction solution was cooled in an ice bath. Next, while the internal temperature of the reaction solution was maintained at 15°C or less, 59.4 g (422.7 mmol) of benzoyl chloride was added dropwise into the reaction solution.

With respect to the mercapto groups in the FSH, the equivalent ratio (chlorine atoms/mercapto groups) of the chlorine atoms in the benzoyl chloride was 1.03.

Next, after the completion of the addition by drops, while the internal temperature of the reaction solution was maintained at 15°C or less, and the reaction solution was stirred for 2 hours.

Thereafter, 550 g of a 10% sodium chloride aqueous solution was added to the reaction solution. Then, the mixture of them was separated to take an organic phase therefrom. Next, the organic phase was washed with 500mL of a 1N dilute hydrochloric acid, further washed with 500 mL of a saturated sodium bicarbonate aqueous solution, and further washed with 500mL of purified water.

A washed solution was passed through an activated alumina (300mesh, for chromatograph, basic). Furthermore, 120 g of toluene was used to cause the reaction product remaining in the alumina to flow out.

An anhydrous sodium sulfate was added to the passed-though washed solution, and stirred to remove the moisture remaining in the washed solution. The sodium sulfate was filtered with a pleated paper filter, thereby producing a toluene diluted solution as a product. Thereafter, the diluted solution was concentrated by an evaporator. In this manner, 5,7-bis(benzoylmercaptomethyl)-1,11-bis(benzoylmercapto)-3,6,9-trithiaundecane (Bz-FSH) was produced as a reaction product.

The Bz-FSH was a light yellowish transparent liquid. The amount of the collected Bz-FSH was 104.8 g. Furthermore, the Bz-FSH had a viscosity (25°C) of 8000mPa·s. Furthermore, the Bz-FSH had a refractive index (nD) of 1.65.

The Bz-FSH was identified by ¹H-NMR (400MHz, CDCl₃) as described below.
¹H-NMR (400MHz, CDCl₃):
δ7.89-8.01 (m, 8H)
δ7.51-7.61 (m, 4H)
δ7.37-7.48 (m, 8H)
δ2.79-3.59 (m, 18H)

### 2. Preparation of Curing Agent

### Preparation Example 1 (Isocyanatethe Curing Agent)

The following commercially available product was prepared as an isocyanate curing agent.

D-110N; trade name TAKENATE D-110N, trimethylol propane (TMP) adduct of xylylene diisocyanate, ethyl acetate solution, solid content concentration of 75% by mass, isocyanate group content of 11.5% by mass, manufactured by Mitsui Chemicals, Inc.

### 3. Synthesis and Main Agent

### Preparation Example 2 (Acrylic Polyol 1)

An acrylic polyol 1 was synthesized by the method below.

First, 120 parts by mass of a polymerized solvent toluene and 140 parts by mass of ethyl acetate were charged into a four-neck flask equipped with a stirrer, a thermometer, a thermometer, and a nitrogen inlet line. While nitrogen was blown into the flask, the internal temperature of the flask was increased to 80±2°C by heating.

Meanwhile, the components below are put into a brown bottle and mixed together. In this manner, a mixed solution was produced.
Toluene 40 parts by mass
NMT-A (1-naphthylmethylacrylate) 80.0 parts by mass
POB-A (m-phenoxybenzylacrylate) 110 parts by mass
HEA (2-hydroxy ethylacrylate) 10.0 parts by mass
PB-O (t-butyl peroxy-2-ethylhexanoate) 2.0 parts by mass

The mixed solution was continuously added dropwise into the above-described flask for 5 hours. After the addition by drops, the solution in the flask was maintained at 80±2°C for 1 hour.

Next, 0.4 parts by mass of PB-O was added into the flask twice every one hour. In this manner, an unreacted component (remaining monomer) was treated. Furthermore, PB-O was added twice, and then the solution in the flask 80±2°C was maintained for 2 hours. In this manner, an acrylic polyol 1 was produced.

The acrylic polyol 1 was a slightly yellowish transparent liquid. The acrylic polyol 1 had a solid content (non-volatile content) concentration of 49.8% by mass. The acrylic polyol 1 had a hydroxyl group value (based on the solid content) of 22.3 mgKOH/g.

### Preparation Example 3 (Acrylic Polyol 2)

The formulation of the mixed solution was changed as follows. Except that, the same operation as Preparation Example 2 was carried out to synthesize an acrylic polyol 2.
Toluene 40 parts by mass
NMT-A (1-naphthylmethylacrylate) 40.0 parts by mass
POB-A (m-phenoxybenzylacrylate) 150 parts by mass
HEA (2-hydroxy ethylacrylate) 10.0 parts by mass
PB-O (t-butyl peroxy-2-ethylhexanoate) 2.0 parts by mass

The acrylic polyol 2 was a yellowish transparent liquid. The acrylic polyol 2 had a solid content (non-volatile content) concentration of 50.4% by mass. The acrylic polyol 2 had a hydroxyl group value (based on the solid content) of 22.4 mgKOH/g.

### Preparation Example 4 (Acrylic Polyol 3)

The formulation of the mixed solution was changed as follows. Except that, the same operation as Preparation Example 2 was carried out to synthesize an acrylic polyol 3.
Toluene 40 parts by mass
POB-A (m-phenoxybenzylacrylate) 190.0 parts by mass
HEA (2-hydroxy ethylacrylate) 10.0 parts by mass
PB-O (t-butyl peroxy-2-ethylhexanoate) 2.0 parts by mass

The acrylic polyol 3 was a yellowish transparent liquid. The acrylic polyol 3 had a solid content (non-volatile content) concentration of 51.0% by mass. The acrylic polyol 3 had a hydroxyl group value (based on the solid content) of 22.4 mgKOH/g.

### Preparation Example 5 (Acrylic Polyol 4)

The following commercially available product was prepared as an acrylic polyol 4.

SG-70L; trade name Teisan Resin SG-70L, carboxy group-containing acrylic polyol, solid content concentration of 12.5% by mass, viscosity of 1600 mPa·s, the sum of the acid value and the hydroxyl group value 5 mgKOH/g, manufactured by Nagase ChemteX Corporation

### 4. Production of Polyurethane Resin

### Examples 7 to 20 and Comparative Examples 1 to 5

In accordance with the formulations shown in Tables 1 and 2, a main agent (cured main agent), and a curing agent and a plasticizer were prepared. A set of them was used as a curable composition. Furthermore, for comparison, dioctyl phthalate (DOP) was prepared as a plasticizer.

Then, a main agent and a curing agent were mixed together so that the equivalent ratio was a value shown in Tables 1 and 2. When the main agent is an acrylic polyol 1 to 3, the equivalent ratio represents the ratio (OH/NCO) of the hydroxyl groups in the main agent with respect to the isocyanate groups in the curing agent.

Furthermore, when the main agent is an acrylic polyol 4 (carboxy group-containing acrylic polyol), the equivalent ratio represents the ratio (OH+COOH/NCO) of the total amount of the hydroxyl groups and carboxy groups in the main agent with respect to the isocyanate groups in the curing agent.

Next, the plasticizer was added to the mixture of the main agent and the curing agent, and mixed together. The adding amount of the plasticizer was adjusted in accordance with Tables 1 and 2. In Tables 1 and 2, the mixing ratio (phr) of the plasticizer represents the part by mass of the plasticizer with respect to 100 parts by mass of the total amount of the solid content of the main agent and the curing agent.

As described above, a mixed solution containing the main agent and the curing agent, and the plasticizer was produced. Next, the above-described mixed solution was applied onto a surface of a polypropylene (PP) substrate. The applying amount was adjusted so that the coating film had a thickness of 200 µm. Then, the coating film was heated at 60°C for 2 hours, and aged at 25°C for 7 days. In this manner, the coating film was cured, thereby producing a cured product. Thereafter, the above-described cured product was released from the PP substrate.

### 5. Evaluations

### (1) Refractive Index (nD)

The refractive index (nD) of the cured product was measured with an Abbe refractometer (manufactured by ATAGO CO.,LTD., DR-M4). The temperature was a room temperature (20°C). Furthermore, the light was a d line (wavelength 587.6 nm).

### (2) Flexibility; Tensile Storage Elastic Modulus (E')

Under the conditions below, the solid viscoelasticity of the cured product was measured. Then, a tensile storage elastic modulus (E') at 25°C was obtained.

Device: RSA-G2 (manufactured by TA Instruments.)
Deformation Mode: tensile mode
Temperature Range: -50°C to 270°C
Temperature Rising Rate: 3°C/min
Frequency: 1Hz
Environment: N₂ environment

### (3) Glass Transition Temperature (Tg)

Under the conditions of the above-described (2), the solid viscoelasticity of the cured product was measured. Then, the tan δ (loss tangent, E''/E') was calculated from the tensile storage elastic modulus (E') and the tensile loss elastic modulus (E''). Then, the temperature at which the loss tangent (tan δ) showed the local maximal value (peak value) was calculated as the glass transition temperature.

### 6. Additive and Evaluations thereof

### Examples 21 to 38

An additive was added to the curable compositions of Examples 7 and 10 to check the weather resistance of the cured product.

More specifically, in accordance with the formulations shown in Tables 3 and 4, a main agent, a curing agent, a plasticizer, and an additive were prepared. A set of them was used as a curable composition. Then, the main agent and the curing agent were mixed together so that the equivalent ratio was a value shown in Table 3.

Next, the plasticizer and the additive were added to the mixture of the main agent and the curing agent. In Table 3, the mixing ratio (phr) of the plasticizer represents the part by mass of the plasticizer with respect to 100 parts by mass of the total amount of the solid content of the mixture of the main agent and the curing agent. Furthermore, in Tables 3 and 4, the mixing ratio (ppm) of the additive represents the mass ratio (ppm) of the additive with respect to the total amount of the solid content (resin solid content (i.e., the total amount of the curable composition and the total amount of the cured product)) of the main agent, the curing agent, the plasticizer, and the additive. In Example 31, an additive was not added.

The types of the additive are described below.
LA-24; trade name ADK STAB LA-24, benzotriazole-based UV absorber, 2-(2H-benzotriazole-2-yl)-4,6-bis(1-methyl-1-phenylethyl)phenol, manufactured by ADEKA
LA-29; trade name ADK STAB LA-29, benzotriazole-based UV absorber, 2-(2H-benzotriazole-2-yl)-4-(1,1,3,3-tetramethylbutyl )phenol, manufactured by ADEKA
LA-32; trade name ADK STAB LA-32, benzotriazole-based UV absorber, 2-(2H-benzotriazole-2-yl)-p-cresol, manufactured by ADEKA
1413; trade name ADK STAB 1413, benzophenone-based UV absorber, [2-hydroxy-4-(octyloxy)phenyl](phenyl)methanone, manufactured by ADEKA
PR-25; trade name Hostavin PR-25, benzylidene-based UV absorber, 2-(4-methoxybenzylidene)propane dioic acid dimethyl, manufactured by CLARIANT
LA-46; trade name ADK STAB LA-46, triazine-based UV absorber, 2-(4,6-diphenyl-1,3,5-triazine-2-yl)-5-[2-(2-ethylhexanoyloxy)ethoxy]phenol, manufactured by ADEKA
LA-72; trade name ADK STAB LA-72, HALS-based light stabilizer, bis(1,2,2,6,6-pentamethyl-4-piperidyl)sebacate, manufactured by ADEKA
AO-60; trade name ADK STAB AO-60, phenol-based antioxidant, pentaerythritol tetrakis[3-(3,5-di-tert-butyl-4-hydroxy phenyl)propionate], manufactured by ADEKA

As described above, a mixed solution containing the main agent and curing agent and the plasticizer and additive was produced. Next, the above-described mixed solution was applied onto a surface of a glass board (D263T-eco, manufactured by SCHOTT, a thickness of 0.21 mm, 50mm×50mm). The applying amount was adjusted so that the coating film had a thickness of 50µm. Then, the coating film was heated at 60°C for 2 hours, and then aged at 25°C for 7 days. In this manner, the coating film was cured, thereby producing a cured product. As a result, a laminate containing the glass board and the cured resin film (hereinafter, a glass board/cured film laminate) was produced.

The YI value and b* (the degree of yellowing before a weather resistance test) of each glass board/cured film laminate was measured with COH-7700 (manufactured by DENSHOKU INDUSTRIES Co.,Ltd., spectrophoto haze meter). The results are shown in Tables 3 and 4.

Thereafter, a glass board/cured film laminate was subjected to a weather resistance test. More specifically, the glass board/cured film laminate was exposed to an ultraviolet ray under the conditions below.

Light Source: Xenon Lamp
Black Panel Temperature: 55°C
Humidity: 55%
Precipitation Condition: N/A
Irradiation Condition: 36.5W/m² (300-400nm)
Filter: Inside/outside = Right Light/Cica-Quarz
Irradiation Surface: Cured Film Side
Irradiation Time: 100 hours

Thereafter, the YI value and b* (the degree of yellowing after the weather resistance test) of each glass board/cured film laminate was measured with COH-7700 (manufactured by DENSHOKU INDUSTRIES Co.,Ltd., spectrophoto haze meter). The results are shown in Tables 3 and 4.

### 7. Production of Cured Acrylic Resin and Evaluations Thereof

### Preparation Example 6 (Acrylic Composition 1)

The components below were put into a brown bottle and mixed together. In this manner, an acrylic composition 1 (a curable compound, an acrylic resin material 1) was produced.
TMP-A (trimethylpropane triacrylate) 40.0 parts by mass
2EHA (2-ethylhexylacrylate) 60.0 parts by mass
Omnirad 184 (alkylphenone-based photo-polymerization initiator, 1-hydroxy cyclohexylphenylketone) 3.0 parts by mass

### Preparation Example 7 (Acrylic Composition 2)

The components below were put into a brown bottle and mixed together. In this manner, an acrylic composition 2 (a curable compound, an acrylic resin material 2) was produced.
TMP-A (trimethylpropane triacrylate) 40.0 parts by mass
POB-A (m-phenoxybenzylacrylate) 60.0 parts by mass
Omnirad 184 (alkylphenone-based photo-polymerization initiator, 1-hydroxy cyclohexylphenylketone) 3.0 parts by mass

### Examples 39 to 46 and Comparative Examples 6 and 7

In accordance with Table 5, a plasticizer and a curable compound are blended, thereby, producing a curable composition (polymerizable composition) was produced.

Next, in a mold formed of a silicon sheet having a thickness of 200 µm, a coating film of the polymerizable composition was sandwiched between release PET films (polyethylene terephthalate films, Purex A3100, manufactured by TOYOBO CO., LTD.). At the time, the release surfaces of the release PET films were brought into contact with the polymerizable composition. In this manner, a laminate 1 (the release PET film/the polymerizable composition/the release PET film) was produced.

Next, the laminate 1 (the release PET film/the polymerizable composition/the release PET film) was sandwiched between glass boards (D263T-eco, manufactured by SCHOTT), and these were fastened with a clip. In other words, the coating film of the polymerizable composition was sandwiched by the glass boards and the release PET films, thereby producing a laminate 2 (the glass board/the release PET film/the polymerizable composition/the release PET film/the glass board).

Thereafter, using an LED light source (365nm), the polymerizable composition of the laminate 2 was irradiated with an active energy ray (a wavelength of 365 nm, an illuminance of 400 mW/cm², an accumulated light intensity of 6000 mJ/cm²). Furthermore, the laminate 2 was heated at 120°C under nitrogen atmosphere for 30 minutes. As a result, the polymerizable composition was radically polymerized by the active energy ray and heat, thereby producing a laminate 3 (the glass board/the release PET film/the cured product/the release PET film/the glass board) including the cured product.

Thereafter, the clip and the glass board were removed, and then the cured product was released from the release PET film. As a result, a cured product was produced. The refractive index (nD), tensile storage elastic modulus (E'), and glass transition temperature (Tg) of the cured product were measured by the above-described methods. The results are shown in Table 5.

Separately from the above, in a mold formed of a silicon sheet having a thickness of 50 µm, the polymerizable composition was sandwiched between glass boards (Eagle-XG, manufactured by CORNIG). In this manner, a laminate 4 (the glass board/the polymerizable composition/the glass board) was produced.

Thereafter, using an LED light source (365 nm), the polymerizable composition of the laminate 4 was irradiated with an active energy ray (a wavelength of 365 nm, an illuminance of 400 mW/cm², an accumulated light intensity of 6000 mJ/cm²). Furthermore, the laminate 4 was heated at 120°C under nitrogen atmosphere for 30 minutes. As a result, the polymerizable composition was radically polymerized by the active energy ray and heat, thereby producing a laminate 5 (the glass board/the cured product/the glass board) including the cured product.

Thereafter, the clip was removed. Then, the YI value and b* (the degree of yellowing before a weather resistance test) of the weather resistance of the laminate 5 (the glass board/the cured product/the glass board) was measured by the same method as the above-described. Furthermore, the laminate 5 (the glass board/the cured product/the glass board) was subjected to a weather resistance by the same method as the above-described. Thereafter, the YI value and b* (the degree of yellowing after the weather resistance test) of the laminate 5 (the glass board/the cured product/the glass board) was measured by the same method as the above-described. The results are shown in Table 5.

### [Table 1]

**Table 1**

| No. | Curable composition | | | | | | Cured product | | |
|---|---|---|---|---|---|---|---|---|---|
| | Plasticizer | | | Curable compound | | | Refractive index | Tg | Storage elastic modulus E' |
| | Type | | Amount | Main agent | Curing agent | Equivalent ratio(OH/NCO) | (nD) | (°C) | (MPa) |
| Ex. 7 | Ex. 1 | Bn-GST | 10phr | Acrylic polyol 1 | D-110N | 1.05 | 1.614 | 38 | 500 |
| Ex. 8 | Ex. 1 | Bn-GST | 10phr | Acrylic polyol 2 | D-110N | 1.05 | 1.608 | 30 | 50 |
| Ex. 9 | Ex. 1 | Bn-GST | 10phr | Acrylic polyol 3 | D-110N | 1.05 | 1.600 | 18 | 2 |
| Ex. 10 | Ex. 2 | Bz-GST | 10phr | Acrylic polyol 1 | D-110N | 1.05 | 1.614 | 37 | 480 |
| Ex. 11 | Ex. 3 | Ac-GST | 10phr | Acrylic polyol 1 | D-110N | 1.05 | 1.607 | 15 | 150 |
| Ex. 12 | Ex. 4 | PA-GST | 10phr | Acrylic polyol 1 | D-110N | 1.05 | 1.612 | 28 | 320 |
| Ex. 13 | Ex. 5 | PP-GST | 10phr | Acrylic polyol 1 | D-110N | 1.05 | 1.610 | 20 | 200 |
| Ex. 14 | Ex. 6 | Bz-FSH | 10phr | Acrylic polyol 1 | D-110N | 1.05 | 1.615 | 40 | 600 |
| Comp. Ex. 1 | - | - | - | Acrylic polyol 1 | D-110N | 1.05 | 1.613 | 50 | 1800 |
| Comp. Ex. 2 | - | - | - | Acrylic polyol 2 | D-110N | 1.05 | 1.606 | 40 | 1500 |
| Comp. Ex. 3 | - | - | - | Acrylic polyol 3 | D-110N | 1.05 | 1.597 | 28 | 40 |
| Comp. Ex. 5 | DOP | DOP | 10phr | Acrylic polyol 1 | D-110N | 1.05 | 1.600 | 30 | 400 |

### [Table 2]

**Table 2**

| No. | Curable composition | | | | | | Cured product | | |
|---|---|---|---|---|---|---|---|---|---|
| | Plasticizer | | | Curable compound | | | Refractive index | Tg | Storage elastic modulus E' |
| | Type | | Amount | Main agent | Curing agent | Equivalent ratio (OH+COOH/NCO) | (nD) | (°C) | (MPa) |
| Ex. 15 | Ex. 1 | Bn-GST | 10phr | Acrylic polyol 4 | D-110N | 1.05 | 1.489 | 1 | 0.8 |
| Ex. 16 | Ex. 1 | Bn-GST | 30phr | Acrylic polyol 4 | D-110N | 1.05 | 1.508 | 0 | 0.8 |
| Ex. 17 | Ex. 2 | Bz-GST | 10phr | Acrylic polyol 4 | D-110N | 1.05 | 1.490 | 0 | 0.8 |
| Ex. 18 | Ex. 2 | Bz-GST | 30phr | Acrylic polyol 4 | D-110N | 1.05 | 1.510 | 0 | 0.7 |
| Ex. 19 | Ex. 2 | Bz-GST | 70phr | Acrylic polyol 4 | D-110N | 1.05 | 1.537 | 0 | 0.6 |
| Ex. 20 | Ex. 3 | Ac-GST | 30phr | Acrylic polyol 4 | D-110N | 1.05 | 1.500 | -1 | 0.6 |
| Comp. Ex. 4 | - | - | - | Acrylic polyol 4 | D-110N | 1.05 | 1.475 | 2 | 0.9 |

### [Table 3]

**Table 3**

| No. | Curable composition | | | | | | | | | Cured product | | | |
|---|---|---|---|---|---|---|---|---|---|---|---|---|---|
| | Plasticizer | | | Curable compound | | | Additive | | | YI value | | b* value | |
| | Type | | Amount | Main agent | Curing agent | Equivalent ratio (OH/NCO) | Type | | Amount (with respect to solid content of resin) | Before weather resistance test | After weather resistance test | Before weather resistance test | After weather resistance test |
| Ex. 21 | Ex. 1 | Bn-GST | 10phr | Acrylic polyol 1 | D-110N | 1.05 | Benzotriazole-based ultraviolet absorber | LA-24 (ADEKA) | 500 ppm | 0.42 | 1.31 | 0.2 | 0.71 |
| Ex. 22 | Ex. 1 | Bn-GST | 10phr | Acrylic polyol 1 | D-110N | 1.05 | Benzotriazole-based ultraviolet absorber | LA-24 (ADEKA) | 1000 ppm | 0.51 | 1.08 | 0.28 | 0.53 |
| Ex. 23 | Ex. 1 | Bn-GST | 10phr | Acrylic polyol 1 | D-110N | 1.05 | Benzotriazole-based ultraviolet absorber | LA-24 (ADEKA) | 10000 ppm | 0.78 | 0.98 | 0.34 | 0.61 |
| Ex. 24 | Ex. 1 | Bn-GST | 10phr | Acrylic polyol 1 | D-110N | 1.05 | Benzotriazole-based ultraviolet absorber | LA-29 (ADEKA) | 500 ppm | 0.25 | 1.11 | 0.13 | 0.6 |
| Ex. 25 | Ex. 1 | Bn-GST | 10phr | Acrylic polyol 1 | D-110N | 1.05 | Benzotriazole-based ultraviolet absorber | LA-32 (ADEKA) | 500 ppm | 0.36 | 1.46 | 0.19 | 0.75 |
| Ex. 26 | Ex. 1 | Bn-GST | 10phr | Acrylic polyol 1 | D-110N | 1.05 | Benzophenone-based ultraviolet absorber | ADK STAB 1413 (ADEKA) | 500 ppm | 0.38 | 1.70 | 0.18 | 0.91 |
| Ex. 27 | Ex. 1 | Bn-GST | 10phr | Acrylic polyol 1 | D-110N | 1.05 | Benzylidene-based ultraviolet absorber | Hostavin PR-25 (CLARIANT) | 500 ppm | 0.37 | 1.23 | 0.24 | 0.65 |
| Ex. 28 | Ex. 1 | Bn-GST | 10phr | Acrylic polyol 1 | D-110N | 1.05 | Triazine-based ultraviolet absorber | LA-46 (ADEKA) | 500 ppm | 0.66 | 1.29 | 0.42 | 0.72 |
| Ex. 29 | Ex. 1 | Bn-GST | 10phr | Acrylic polyol 1 | D-110N | 1.05 | HALS-based light stabilizer | LA-72 (ADEKA) | 500 ppm | 0.35 | 1.54 | 0.19 | 0.87 |
| Ex. 30 | Ex. 1 | Bn-GST | 10phr | Acrylic polyol 1 | D-110N | 1.05 | Phenol-based antioxidant | AO-60 (ADEKA) | 500 ppm | 0.40 | 2.46 | 0.24 | 1.56 |
| Ex. 31 | Ex. 1 | Bn-GST | 10phr | Acrylic polyol 1 | D-110N | 1.05 | - | - | - | 0.38 | 11.50 | 0.19 | 5.75 |

### [Table 4]

**Table 4**

| No. | Curable composition | | | | | | | | | Cured product | | | |
|---|---|---|---|---|---|---|---|---|---|---|---|---|---|
| | Plasticizer | | | Curable compound | | | Additive | | | YI value | | b* value | |
| | Type | | Amount | Main agent | Curing agent | Equivalent ratio (OH/NCO) | Type | | Amount (with respect to solid content of resin) | Before weather resistance test | After weather resistance test | Before weather resistance test | After weather resistance test |
| Ex. 32 | Ex. 2 | Bz-GST | 10phr | Acrylic polyol 1 | D-110N | 1.05 | Benzotriazole-based ultraviolet absorber | LA-24 (ADEKA) | 500 ppm | 0.41 | 1.42 | 0.18 | 0.42 |
| Ex. 33 | Ex. 2 | Bz-GST | 10phr | Acrylic polyol 1 | D-110N | 1.05 | Benzotriazole-based ultraviolet absorber | LA-29 (ADEKA) | 500 ppm | 0.35 | 1.25 | 0.17 | 0.53 |
| Ex. 34 | Ex. 2 | Bz-GST | 10phr | Acrylic polyol 1 | D-110N | 1.05 | Benzophenone-based ultraviolet absorber | ADK STAB 1413 (ADEKA) | 500 ppm | 0.37 | 1.19 | 0.2 | 0.44 |
| Ex. 35 | Ex. 2 | Bz-GST | 10phr | Acrylic polyol 1 | D-110N | 1.05 | Benzylidene-based ultraviolet absorber | Hostavin PR-25 (CLARIANT) | 500 ppm | 0.44 | 1.45 | 0.19 | 0.38 |
| Ex. 36 | Ex. 2 | Bz-GST | 10phr | Acrylic polyol 1 | D-110N | 1.05 | Triazine-based ultraviolet absorber | LA-46 (ADEKA) | 500 ppm | 0.32 | 1.44 | 0.15 | 0.51 |
| Ex. 37 | Ex. 2 | Bz-GST | 10phr | Acrylic polyol 1 | D-110N | 1.05 | HALS-based light stabilizer | LA-72 (ADEKA) | 500 ppm | 0.38 | 1.34 | 0.22 | 0.58 |
| Ex. 38 | Ex. 2 | Bz-GST | 10phr | Acrylic polyol 1 | D-110N | 1.05 | Phenol-based antioxidant | AO-60 (ADEKA) | 500 ppm | 0.36 | 1.38 | 0.17 | 0.44 |

### [Table 5]

**Table 5**

| No. | Resin composition | | | | | | Cured product | | | | | | |
|---|---|---|---|---|---|---|---|---|---|---|---|---|---|
| | Plasticizer | | | Curable compound | | | Refractive index | Tg | Storage elastic modulus E' | YI value | | b* value | |
| | Type | | Amount | Type | | Amount | (nD) | (°C) | (MPa) | Before weather resistance test | After weather resistance test | Before weather resistance test | After weather resistance test |
| Ex. 39 | Ex. 2 | Bz-GST | 10phr | Prep. Ex. 6 | Acrylic composition 1 | 100 parts by mass | 1.509 | 25 | 90 | 0.4 | 10.6 | 0.2 | 5.8 |
| Ex. 40 | Ex. 3 | Ac-GST | 10phr | Prep. Ex. 6 | Acrylic composition 1 | 100 parts by mass | 1.504 | 15 | 30 | 0.4 | 0.5 | 0.2 | 0.3 |
| Ex. 41 | Ex. 4 | PA-GST | 10phr | Prep. Ex. 6 | Acrylic composition 1 | 100 parts by mass | 1.507 | 23 | 80 | 0.4 | 0.9 | 0.2 | 0.6 |
| Ex. 42 | Ex. 5 | PP-GST | 10phr | Prep. Ex. 6 | Acrylic composition 1 | 100 parts by mass | 1.505 | 18 | 40 | 0.4 | 0.5 | 0.2 | 0.3 |
| Ex. 43 | Ex. 2 | Bz-GST | 10phr | Prep. Ex. 7 | Acrylic composition 2 | 100 parts by mass | 1.585 | 32 | 150 | 0.8 | 19.3 | 0.5 | 10.8 |
| Ex. 44 | Ex. 3 | Ac-GST | 10phr | Prep. Ex. 7 | Acrylic composition 2 | 100 parts by mass | 1.576 | 40 | 90 | 0.7 | 1.9 | 0.5 | 1.1 |
| Ex. 45 | Ex. 4 | PA-GST | 10phr | Prep. Ex. 7 | Acrylic composition 2 | 100 parts by mass | 1.585 | 35 | 120 | 0.9 | 2.3 | 0.5 | 1.5 |
| Ex. 46 | Ex. 5 | PP-GST | 10phr | Prep. Ex. 7 | Acrylic composition 2 | 100 parts by mass | 1.578 | 37 | 100 | 0.9 | 2.1 | 0.5 | 1.2 |
| Comp. Ex. 6 | - | - | - | Prep. Ex. 6 | Acrylic composition 1 | 100 parts by mass | 1.492 | 49 | 500 | 0.4 | 0.4 | 0.2 | 0.2 |
| Comp. Ex. 7 | - | - | - | Prep. Ex. 7 | Acrylic composition 2 | 100 parts by mass | 1.574 | 68 | 2000 | 0.4 | 1.8 | 0.2 | 1.1 |

While the illustrative embodiments of the present invention are provided in the above description, such is for illustrative purpose only and it is not to be construed as limiting in any manner. Modification and variation of the present invention that will be obvious to those skilled in the art is to be covered by the following claims.

### Industrial Applicability

The compound, additive, plasticizer, curable composition, adhesive, cured product, and pressure-sensitive adhesive of the present invention are preferably used in the field of coating agents, the field of paints, the field of adhesives, the field of films, and the field of pressure-sensitive adhesives.

## Claims

1. A compound represented by the following general formula (1).
General Formula (1);
[Chem. 1] Chem. 1
A-(SXR)ₙ (1)
(In the formula (1), the A represents an n-valent organic group including a sulfur atom. The n represents an integer of 3 or more. The S represents a sulfur atom. The X represents a single bond or a carbonyl group. The Xs may be the same or different from each other. The R represents an aliphatic hydrocarbon group, an aromatic hydrocarbon group, or an araliphatic hydrocarbon group. The Rs may be the same or different from each other.)

2. The compound according to claim 1,
wherein the A represents an organic group including a sulfur atom and a main-group-element atom (other than a sulfur atom and a hydrogen atom),
wherein in the A, a ratio of the number of the sulfur atoms is more than 20% with respect to a sum of the number of the sulfur atoms and the number of the main-group-element atoms (other than a sulfur atom and a hydrogen atom).

3. The compound according to claim 1, being represented by the following general formula (2) or the following general formula (3). 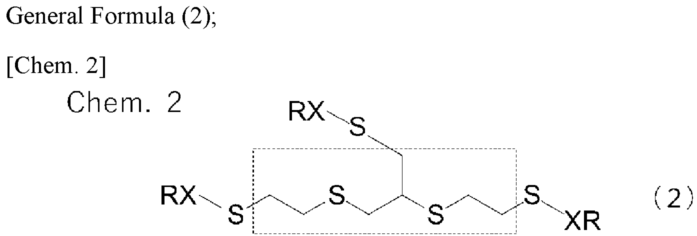 (In the formula (2), the S, X, and R represent the same meanings as the S, X, and R of the formula (1). In the formula (2), a portion surrounded by a dotted line represents an organic group A (n=3) of the formula (1).) 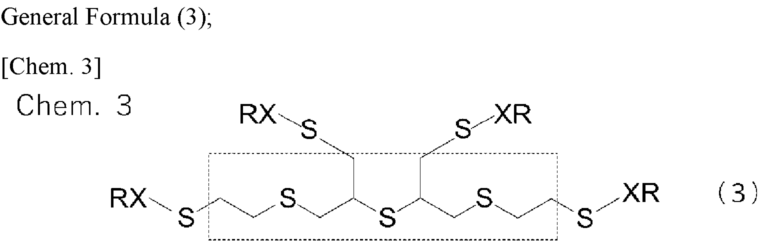 (In the formula (3), the S, X, and R represent the same meanings as the S, X, and R of the formula (1). In the formula (3), a portion surrounded by a dotted line represents an organic group A (n=4) of the formula (1).)

4. The compound according to claim 1,
wherein the R represents a methyl group, a phenyl group, or a benzyl group.

5. The compound according to claim 1,
wherein the X represents a carbonyl group, and the R represents an araliphatic hydrocarbon group.

6. An additive comprising: the compound according to claim 1.

7. A plasticizer comprising: the compound according to claim 1.

8. A curable composition comprising: the plasticizer according to claim 7 and a curable compound.

9. The curable composition according to claim 8, further comprising:
at least one additive selected from the group consisting of an ultraviolet absorber, a light-resistant stabilizer, and an antioxidant,
wherein a ratio of the additive is 10×10⁻⁶ parts by mass or more and 10000×10⁻⁶ parts by mass or less with respect to 1 part by mass of a total amount of the curable composition.

10. An adhesive comprising: the curable composition according to claim 8.

11. A cured product comprising: the plasticizer according to claim 7 and a cured resin.

12. The cured product according to claim 11, having a refractive index of 1.60 or more and a tensile storage elastic modulus of 100 MPa or less.

13. The cured product according to claim 11, further comprising:
at least one additive selected from the group consisting of an ultraviolet absorber, a light-resistant stabilizer, and an antioxidant,
wherein a ratio of the additive is 10×10⁻⁶ parts by mass or more and 10000×10⁻⁶ parts by mass or less with respect to 1 part by mass of a total amount of the cured product.

14. The cured product according to claim 11,
wherein the cured resin includes a reaction product containing a main agent containing an acrylic polyol and a curing agent containing a polyisocyanate.

15. The cured product according to claim 11,
wherein the cured resin includes a cured acrylic resin.

16. A pressure-sensitive adhesive comprising: the cured product according to claim 11.
